# EUROPEAN PATENT APPLICATION

(11) **EP 0 861 838 A2**
(43) Date of publication of application: **02.09.1998**
(21) Application number: 98301367.3
(22) Date of filing: 25.02.1998
(51) Int. Cl.: C07D 413/06, C07D 419/06, C07D 303/46, C07D 303/38, C07D 303/40, C07D 405/12, C07F 7/18, C07C 271/22

(54) **Process and intermediates for preparing cryptophycin compounds**

(30) Priority: 26.02.1997 US 39116 P; 26.02.1997 US 39114 P
(71) Applicant: ELI LILLY AND COMPANY, Indianapolis, Indiana 46285 (US); UNIVERSITY OF HAWAII, Honolulu, HI 96822 (US); Wayne State University, Detroit, Michigan 48202 (US)
(72) Inventor: Patel, Vinod Francis, Carmel, Indiana 46032 (US); Hoard, David Warren, Greenwood, Indiana 46142 (US); Moher, Eric David, Indianapolis, Indiana 46237 (US)
(74) Representative: Hudson, Christopher Mark

(57) **Abstract**

Cryptophycin compounds may be prepared utilizing an epoxidation step early in the synthetic process under the conditions set forth herein. This invention also relates to novel intermediates generated by this early epoxidation process.

## Description

### BACKGROUND OF THE INVENTION

Neoplastic diseases, characterized by the proliferation of cells not subject to the normal control of cell growth, are a major cause of death in humans and other mammals. Clinical experience in cancer chemotherapy has demonstrated that new and more effective drugs are desirable to treat these diseases. Such clinical experience has also demonstrated that drugs which disrupt the microtubule system of the cytoskeleton can be effective in inhibiting the proliferation of neoplastic cells.

Cryptophycin compounds can now be prepared using a total synthetic process; however, many of the useful cryptophycin compounds contain an acid labile epoxide group. Barrow, R.A. et al., *J. Am. Chem. Soc.* 117, 2479 (1995). Applicants have discovered that the beta-epoxide can be particularly desired. However, in the Barrow et al. synthesis of some of the cryptophycin compounds of formula (I) below, the epoxidation is performed in the last step which provides only a 2:1 selectivity for the desired epoxide. Furthermore, the diastereomers are difficult to separate at this stage. While it would be desirable to epoxidize an earlier intermediate in the process, epoxides are sensitive to a number of reaction conditions.

The present invention provides a much desired novel and efficient method for preparing cryptophycin compounds having an epoxide functionality. The epoxidation takes place at an early step in the overall synthetic process allowing for easier separation of diastereomers and for higher selectivity. Further, epoxidating at an earlier step increases overall efficiency of the process by preserving materials, reducing costs and enhancing throughput.

### BRIEF SUMMARY OF THE INVENTION

The present invention relates to a process for preparing a compound of the formula 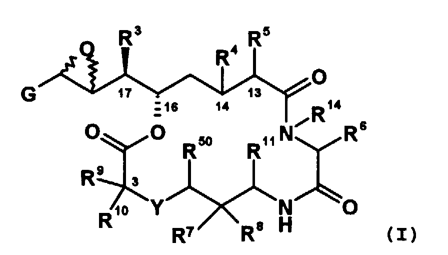
wherein
G is C₁-C₁₂ alkyl, C₂-C₁₂ alkenyl, C₂-C₁₂ alkynyl, or Ar;
Ar is an aromatic or heteroaromatic group or a substituted aromatic or heteroaromatic group;
R³ is C₁-C₆ alkyl;
R⁴ and R⁵ are each hydrogen; or R⁴ and R⁵ taken together form a second bond between C-13 and C-14;
R⁷ and R⁸ are each independently hydrogen or C₁-C₆ alkyl; or
R⁷ and R⁸ taken together form a cyclopropyl or cyclobutyl ring;
R⁹ is hydrogen, C₁-C₆ alkyl, C₂-C₆ alkenyl, C₂-C₆ alkynyl, -(CH₂)ₘ-(C₃-C₅)cycloalkyl or benzyl, wherein m is the integer one to three;
R¹⁰ is hydrogen or C₁-C₆ alkyl;
R¹¹ is hydrogen, C₁-C₆ alkyl, phenyl or benzyl;
R¹⁴ is hydrogen or C₁-C₆ alkyl;
R⁵⁰ is hydrogen or (=O);
Y is CH, O, NR¹², S, SO, SO₂, wherein R¹² is H or C₁-C₃ alkyl;
R⁶ is C₁-C₆ alkyl, substituted (C₁-C₆)alkyl, (C₃-C₈)cycloalkyl, substituted (C₃-C₈)cycloalkyl, a heteroaromatic or substituted heteroaromatic group or a group of formula (IA), (IB) or (IC): 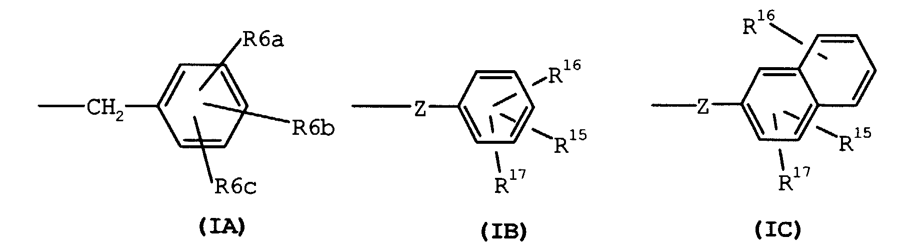
**R**^{**6a**}**, R**^{**6b**}**,** and **R**^{**6c**} independently are H, (C₁-C₆)alkyl, halo NR¹⁸R¹⁹ or OR¹⁸;
**R**^{**15**}**, R**^{**16**}**,** and **R**^{**17**} independently are hydrogen, halo, (C₁-C₆)alkyl, OR¹⁸, O-aryl, NH₂, NR¹⁸R¹⁹, NO₂, OPO₄H₂, (C₁-C₆ alkoxy)phenyl, S-benzyl, CONH₂, C0₂H, PO₃H₂, SO₂R²³, or Z';
**R**^{**18**} and **R**^{**19**} independently are hydrogen or C₁-C₆ alkyl;
**R**^{**23**} is hydrogen or (C₁-C₃)alkyl;
**Z** is -(CH₂)ₙ- or (C₃-C₅)cycloalkyl;
**n** is 0, 1, or 2; and
**Z'** is an aromatic or substituted aromatic group; or a pharmaceutically acceptable salt thereof;

comprising deprotecting a compound of the formula 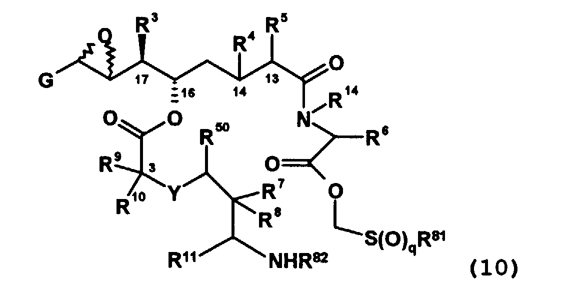 wherein G, R³, R⁵, R⁶, R⁷, R⁸, R⁹, R¹⁰, R¹¹, R¹⁴, R⁵⁰ and Y are as defined above; q is an integer 1 or 2; R⁸¹ is C₁-C₆ alkyl, C₃-C₈ cycloalkyl, phenyl or benzyl; and R⁸² is a base labile protecting group, with a deprotecting agent to form a compound of the formula 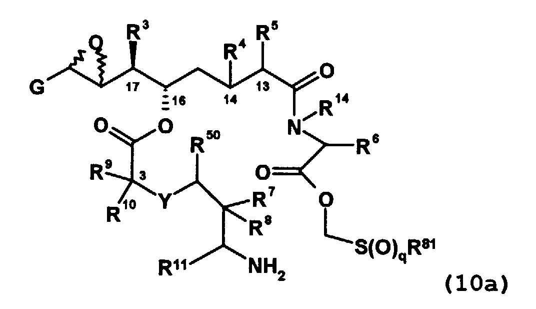 wherein G, R³, R⁵, R⁶, R⁷, R⁸, R⁹, R¹⁰, R¹¹, R¹⁴, R⁵⁰, Y, q and R⁸¹ are as defined above; optionally contacting the compound of formula (9c) with a cyclizing agent to form a compound of formula (I); and optionally forming a pharmaceutically acceptable salt thereof.

In addition, the present invention relates to a process for preparing a compound of formula (I) comprising the steps of:
(a) epoxidizing a compound of the formula 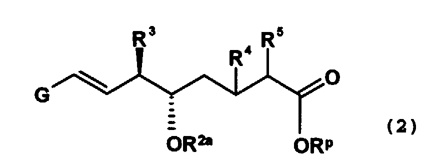 wherein G, R³, R⁴ and R⁵ are as defined above; R^{2a} is hydrogen or tri(C₁-C₆ alkyl)silyl; and R^{p} is hydrogen or a suitable activatable carboxy protecting group; with an epoxidizing agent to form a compound of the formula 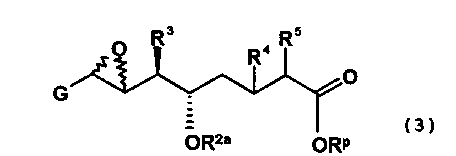 wherein G, R³, R⁴, R⁵,R^{2a} and R^{p} are as defined above, provided that R^{2a} and R^{p} are not both hydrogen;
(b) coupling the compound of formula (3) with an amino acid of the formula 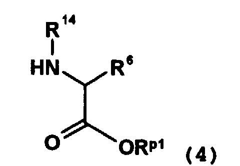 wherein R⁶ and R¹⁴ are as defined above and R^{p1} is hydrogen or C₁-C₆ alkyl; further in the presence of a silylating agent when R¹⁴ and R^{p1} are hydrogen; to yield a compound of the formula 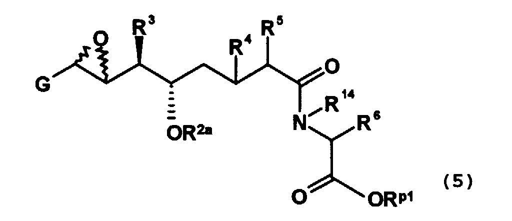 wherein G, R³, R⁴, R⁵, R^{2a}, R^{p1} R⁶ and R¹⁴ are as defined above;
(c) deprotecting the compound of formula (5) with a suitable alkoxy deprotecting agent and further carboxy-deprotecting the compound of formula (5) when R^{p1} is C₁-C₆ is alkyl, with a suitable base to form a compound of the formula 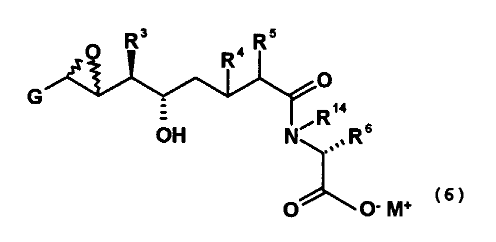 wherein G, R³, R⁴, R⁵, R⁶ and R¹⁴ are as defined above and M⁺ is a cation;
(d) contacting a compound of formula (6) with a thioester forming agent to form a compound of the formula 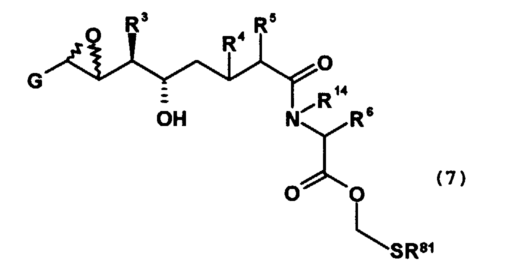 wherein G, R³, R⁴, R⁵, R⁶, R¹⁴ and R⁸¹ are as defined above;
(e) coupling a compound of formula (7) with a compound of the formula 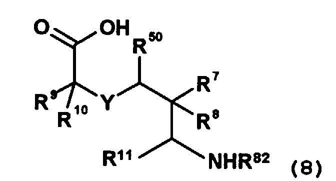 wherein R⁷, R⁸, R⁹, R¹⁰, R¹¹, R⁵⁰ and R⁸² are as defined above, to form a compound of the formula 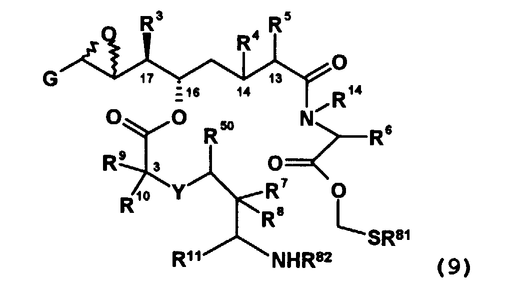 wherein G, R³, R⁴, R⁵, R⁶, R⁷, R⁸, R⁹, R¹⁰, R¹¹, R¹⁴, R⁵⁰ R⁸¹, R⁸² and Y are as defined above;
(f) oxidizing a compound of formula (9) with an oxidizing agent to form a compound of the formula 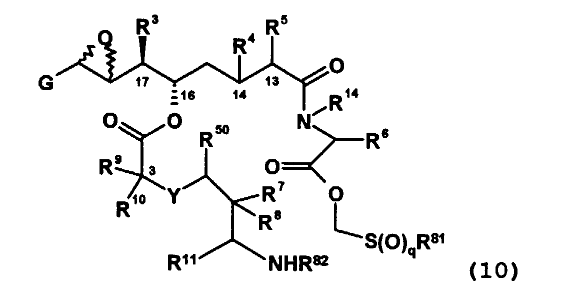 wherein G, R³, R⁴, R⁵, R⁶, R⁷, R⁸, R⁹, R¹⁰, R¹¹, R¹⁴, R⁵⁰, R⁸¹ and R⁸², Y and q are as defined above;
(g) deprotecting a compound of formula (10) with a suitable deprotecting agent to form a compound of the formula 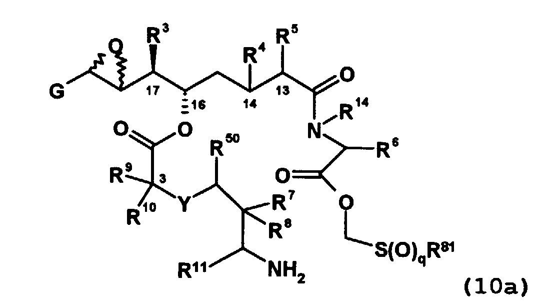 wherein G, R³, R⁵, R⁶, R⁷, R⁸, R⁹, R¹⁰, R¹¹, R¹⁴, R⁵⁰, Y and R⁸¹ are as defined above; and optionally contacting a compound of formula (10a) with a cyclizing agent to form a compound of formula (I); and
(h) optionally forming a pharmaceutically acceptable salt of a compound of formula (I).

In addition, the present invention relates to a compound of the formula 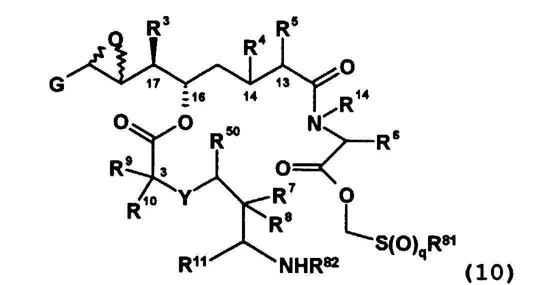 wherein G, R³, R⁴, R⁵, R⁶, R⁷, R⁸, R⁹, R¹⁰, R¹¹, R¹⁴, R⁵⁰, R⁸¹ and R⁸², Y and q are as defined above; or a pharmaceutically acceptable salt thereof; useful as an intermediate for making anti-neoplastic and/or anti-fungal agents.

In addition, the present invention relates to a compound of the formula 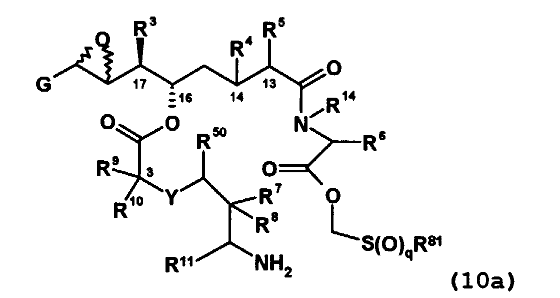 wherein G, R³, R⁵, R⁶, R⁷, R⁸, R⁹, R¹⁰, R¹¹, R¹⁴, R⁵⁰, Y and R⁸¹ are as defined above or a pharmaceutically acceptable salt thereof; useful as an intermediate for making anti-neoplastic and/or anti-fungal agents.

In addition, the present invention relates to a compound of the formula 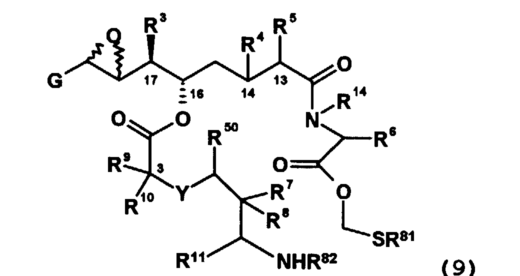 wherein G, R³, R⁴, R⁵, R⁶, R⁷, R⁸, R⁹, R¹⁰, R¹¹, R¹⁴, R⁵⁰ R⁸¹, R⁸² and Y are as defined above; or a pharmaceutically acceptable salt thereof; useful as an intermediate for making anti-neoplastic and/or anti-fungal agents.

In addition, the present invention relates to a compound of the formula 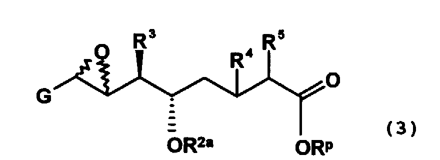 wherein G, R³, R⁴, R⁵, R^{2a} and R^{p} are as defined above, provided that R^{2a} and R^{p} are not both hydrogen; useful as an intermediate for making anti-neoplastic and/or anti-fungal agents.

Further, the present invention relates to a compound of the formula 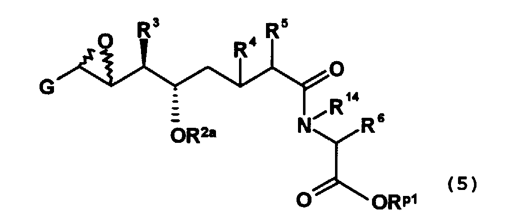 wherein G, R³, R⁴, R⁵, R^{2a}, R^{p1} R⁶ and R¹⁴ are as defined above; useful as an intermediate for making anti-neoplastic and/or anti-fungal agents.

Further, the present invention relates to a compound of the formula 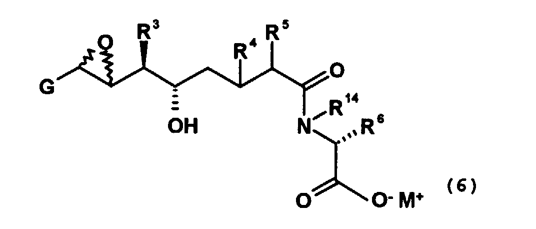 wherein G, R³, R⁴, R⁵, R⁶ and R¹⁴ are as defined above and M⁺ is a cation; useful as an intermediate for making anti-neoplastic and/or anti-fungal agents.

In addition, the invention relates to a process for preparing a compound of formula (I) comprising deprotecting a compound of formula (9) with a deprotecting agent to form a compound of the formula 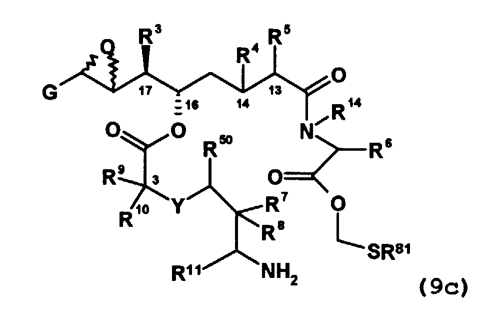 G, R³, R⁴, R⁵, R⁶, R⁷, R⁸, R⁹, R¹⁰, R¹¹, R¹⁴, R⁵⁰ and Y are as defined above; optionally contacting a compound of formula (9c) with a cyclizing agent to form a compound of formula (I); and optionally forming a pharmaceutically acceptable salt thereof.

### DETAILED DESCRIPTION OF THE INVENTION

As used in the application:
(a) the designation " 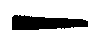 " refers to a bond that protrudes forward out of the plane of the page;
(b) the designation " 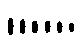 " refers to a bond that protrudes backward out of the plane of the page; and
(c) the designation " 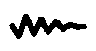 " refers to a bond for which the stereochemistry is not designated.

As used herein, the term "pharmaceutically acceptable salt" refers to either acid addition salts or base addition salts.

The expression "pharmaceutically acceptable acid addition salt" is intended to apply to any non-toxic organic or inorganic acid addition salt of the compounds of formula I or any of its intermediates. Illustrative inorganic acids which form suitable salts include hydrochloric, hydrobromic, sulphuric, and phosphoric acid and acid metal salts such as sodium monohydrogen orthophophate, and potassium hydrogen sulfate. Illustrative organic acids which form suitable salts include the mono-, di- and tricaboxylic acids. Illustrative of such acids are for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phenoxy-benzoic, and sulfonic acids such as p-toluenesulfonic acid, methane sulfonic acid and 2-hydroxyethane sulfonic acid. Such salts can exist in either hydrated or substantially anhydrous form.

The expression "pharmaceutically acceptable basic addition salts" is intended to apply to any non-toxic organic or inorganic basic addition salts of the compounds of formula I or any of its intermediates. Illustrative bases which form suitable salts include alkali metal or alkaline-earth metal hydroxides such as sodium, potassium, calcium, magnesium or barium hydroxides; ammonia and aliphatic, cyclic or aromatic organic amines such as methylamine, dimethylamine, trimethylamine, diethylamine, triethylamine, isopropyldiethylamine, pyridine and picoline.

As used herein, the term "C₁-C₁₂ alkyl" refers to a saturated straight or branched chain hydrocarbon group of from one to twelve carbon atoms. Included within the scope of this term are methyl, ethyl, n-propyl, isopropyl, n-butyl, isobutyl, sec-butyl, tert-butyl, pentyl, neopentyl, 2-methylbutyl, 3-methylbutyl, hexyl, heptyl, octyl, nonyl, decyl and the like. Included within the term is the term "C₁-C₆ alkyl" which refers to a saturated, unsaturated, straight or branched chain hydrocarbon radical of from one to six carbon atoms. Included within the scope of this term are methyl, ethyl, n-propyl, isopropyl, n-butyl, isobutyl, sec-butyl, tert-butyl, pentyl, neopentyl, 2-methylbutyl, 3-methylbutyl, hexyl and the like. Included within the terms "C₁-C₁₂ alkyl" and "C₁-C₆ alkyl" is the terms "C₁-C₃ alkyl" which refers to a saturated, unsaturated, straight or branched chain hydrocarbon radical of from one to three carbon atoms. Included within the scope of this term are methyl, ethyl, isopropyl, and the like.

"Substituted (C₁-C₆)alkyl" refers to a C₁-C₆ alkyl group that may include up to three (3) substituents containing one or more heteroatoms. Examples of such substituents are OH, NH₂, CONH₂, C0₂H, P0₃H₂ and S0₂R²¹ wherein **R**^{**21**} is hydrogen, C₁-C₃ alkyl or aryl.

The term "(C₃-C₈)cycloalkyl" refers to a saturated C₃-C₈ cycloalkyl group. Included within this group are cyclopropyl, cyclobutyl, cyclohexyl, cyclooctyl, and the like. A "substituted (C₃-C₈)cycloalkyl group" refers to a (C₃-C₈)cycloalkyl group having up to three C₁-C₃ alkyl, halo, or OR²¹ substituents. The substituents may be attached at any available carbon atom. Cyclohexyl is an especially preferred cycloalkyl group. The term "-(CH₂)ₘ-(C₃-C₅) cycloalkyl" where m is an integer one, two or three refers to a cyclopropyl, cyclobutyl or cyclopentyl ring attached to a methylidene, ethylidene or propylidene substituent.

The term "C₂-C₁₂ alkenyl" refers to an unsaturated straight or branched chain hydrocarbon radical of from two to twelve carbon atoms and having from one to three triple bonds. Included within the scope of this term are ethenyl, propenyl, isopropenyl, n-butenyl, isobutenyl, pentenyl, 2-methylbutenyl, 3-methylbutenyl, hexenyl, octenyl, nonenyl, decenyl and the like. It is especially preferred that alkenyl have only one double bond.

The term "C₂-C₁₂ alkynyl" refers to an unsaturated straight or branched chain hydrocarbon radical of from two to twelve carbon atoms and having from one to three double bonds. Included within the scope of this term are ethynyl, propynyl, isopropynyl, 2-methypropynyl, hexynyl, decynyl, and the like. It is particularly preferred that alkynyl has only one triple bond.

The term "C₁-C₆ alkoxy" refers to a straight or branched alkoxy group containing from one to six carbon atoms, such as methoxy, ethoxy, n-propoxy, isopropoxy, n-butoxy, isobutoxy, pentoxy, 2-methylpentoxy, and the like. The term "(C₁-C₆ alkoxy)phenyl" refers to a phenyl group substituted with a C₁-C₆ alkoxy group at any available carbon on the phenyl ring.

The term "halo" refers to chloro, bromo, fluoro, or iodo.

The terms "aromatic group" and "heteroaromatic group" refer to common aromatic rings having 4n + 2 pi electrons in a monocyclic or bicyclic conjugated system. The term "aryl" refers to an aromatic group, and the term "aralkyl" refers to an aryl(C₁-C₆-alkyl) group. Examples of aromatic groups are phenyl, benzyl and naphthyl. Heteroaromatic groups will contain one or more oxygen, nitrogen and/or sulfur atoms in the ring. Examples of heteroaromatic groups include furyl, pyrrolyl, thienyl, pyridyl and the like. When the aromatic or heteroaromatic groups are substituted, they may have from one to three independently selected C₁-C₆ alkyl, C₁-C₆-alkoxy or halo, substituents. The aromatic groups may be further substituted with trifluoromethyl, COOR⁵⁷ (wherein R⁵⁷ is hydrogen or C₁-C₆ alkyl), PO₃H, SO₃H, SO₂R⁵⁷, N(R⁵⁹) (R⁶⁰) (wherein R⁵⁹ is hydrogen or C₁-C₆ alkyl and R⁶⁰ is hydrogen, C₁-C₆ alkyl, BOC or FMOC), -CN, -NO₂, -OR⁵⁷, -CH₂OC(O) (CH₂)ₘ,NH₂ (wherein m' is an integer 1 to 6) or -CH₂-O-Si(R⁵⁷) (R⁵⁸) (R⁵⁹) (wherein R⁵⁸ is hydrogen or C₁-C₆ alkyl). Especially preferred substituents for the aromatic groups include methyl, halo, N(R⁵⁹)(R⁶⁰), and -OR⁵⁷. The substituents may be attached at any available carbon atom.

Especially preferred heterocyclic or substituted heterocyclic groups include 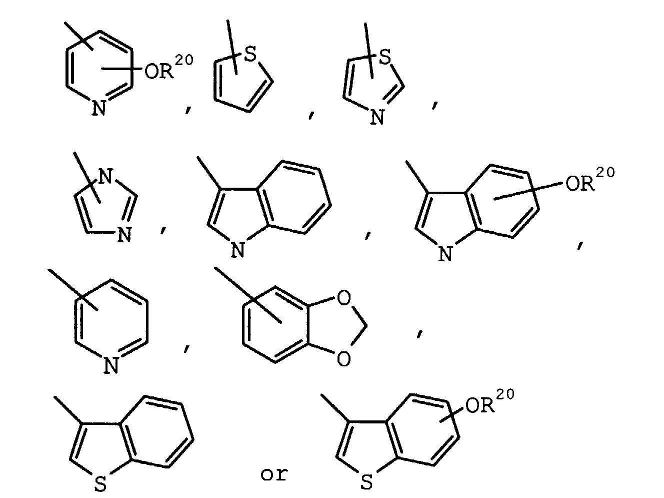 wherein **R**^{**20**} is hydrogen or C₁-C₆ alkyl.

The term "aryl" refers to an aromatic group of from 6 to 12 carbon atoms, such as phenyl or naphthyl groups wherein said groups are optionally substituted with one, two or three substituents selected from the group consisting of C₁-C₄ alkyl, halo-substituted C₁-C₄ alkyl, halogen or C₁-C₄ alkoxy. The terms "lower alkoyl group" or "C₁-C₅ alkoxy" refers to an alkyloxy radical made up of an oxygen radical bearing a saturated straight or branched chain hydrocarbyl radical of one to five carbon atoms and specifically includes methoxy, ethoxy, propyloxy, isopropyloxy, n-butyloxy, isobutyloxy, sec-butyloxy, tertiary butyloxy, pentyloxy and the like. Specifically included with the scope of the term "aryl" are phenyl, p-toluoyl, p-methoxyphenyl, p-chlorophenyl, naphthyl and the like.

As used herein, the term "heteroaryl" refers to a substituted or unsubstituted heteroaromatic radical which contains one or more non-carbon substituents within the ring, said substituents selected from oxygen, nitrogen or sulfur. The total number of carbon atoms and non-carbon atoms in the ring range from four to twelve atoms. Specifically included with the scope of the term "heteroaryl" are monocyclic conjugated systems such as furyl, pyrrolyl, thienyl, pyridyl, and the like and bicyclic conjugated systems such as indole.

As used herein "epoxide ring" means a three-membered ring whose backbone consists of two carbons and an oxygen atom. As used herein, "aziridine ring" means a three-membered ring whose backbone consists of two carbon atoms and a nitrogen atom. As used herein "sulfide ring" means a three-membered ring whose backbone consists of two carbon atoms and a sulfur atom. As used herein "episulfide ring" means a three-membered ring whose backbone consists of two carbon atoms and a sulfur atom. As used herein "sulfate group" means a five membered ring consisting of a carbon-carbon-oxygen-sulfur-oxygen backbone with two additional oxygen atoms connected to the sulfur atom. As used herein "cyclopropyl ring" means a three member ring whose backbone consists of three carbon atoms. As used herein, "monoalkylphosphate ring" means a five membered ring consisting of a carbon-carbon-oxygen-phosphorous-oxygen backbone with two additional oxygen atoms, one of which bears a lower alkyl group, connected to the phosphorous atom.

As used herein, the term "(=O)" in combination with the carbon on the ring to which it is attached refers to a carbonyl group of the formula

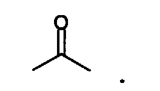

The term "O-aryl" refers to an aryloxy or an aryl group bonded to an oxy moiety.

As used herein, the term "TBS" refers to tertbutyldimethylsilyl represented by the formula

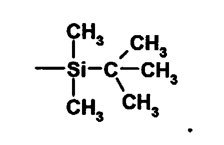

As used herein, the term "NHS" refers to N-hydroxysuccinimide represented by the formula

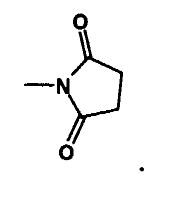

As used herein the term "Ph" refers to a phenyl moiety.

As used herein the term "base labile amino protecting group" refers to common amino protecting groups which are known to be base labile. The artisan can consult common works such as Greene, T.W. "Protecting Groups in Organic Synthesis", Wiley (New York, 1981). See particularly Chapter 7 of Greene. An especially preferred base labile amino protecting group is fluorenylmethoxycarbonyl (Fmoc).

The term "suitable activatable carboxy protecting group" refers to carboxy protecting groups containing activatable ester substituents and are known by one of ordinary skill in the art and disclosed by Greene, T.W., supra. Suitable carboxy protecting groups are those which are activatable ester substituents including N-hydroxy-succinimide, N-hydroxysulfosuccinimide and salts thereof, 2-nitrophenyl, 4-nitrophenyl, 2,4-dichlorophenyl, and the like. An especially preferred activatable carboxy protecting group is N-hydroxy-succinimide (NHS).

A general synthetic procedure is set forth in Scheme A. In Scheme A, all substituents unless otherwise indicated, are as previously defined. Reagents, techniques, and procedures used in Scheme A are well known and appreciated by one of ordinary skill in the art.

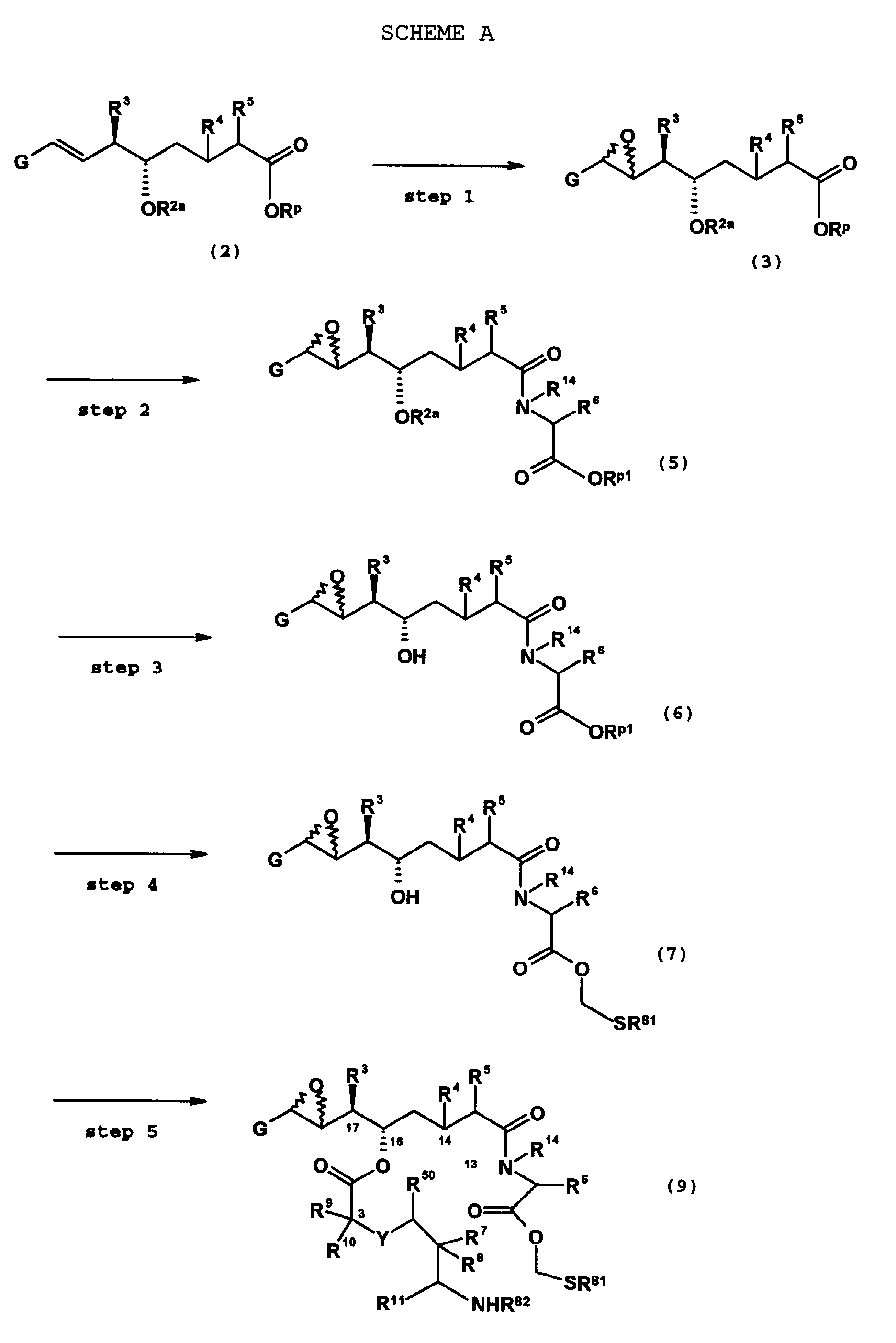

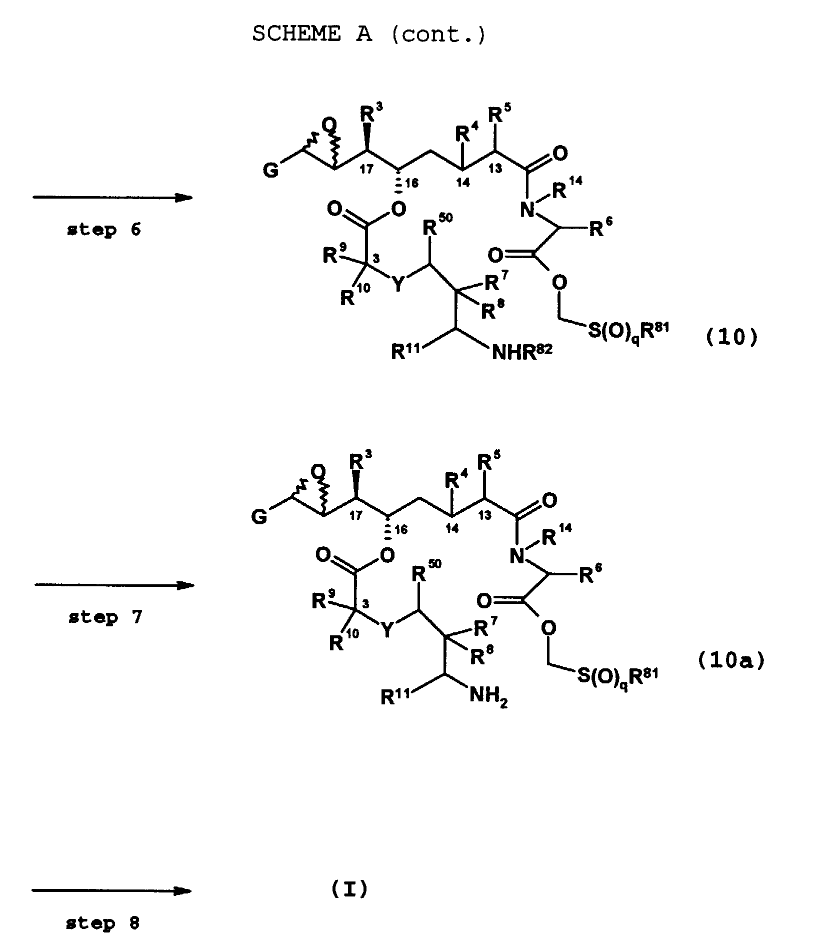

In Scheme A, step 1, an alkene of formula (2) is epoxidized with an epoxidizing agent to form an epoxide of formula (3).

The compound of formula (2) may be epoxidized non-selectively using a suitable epoxidizing agent. An "epoxidizing agent" is an agent capable of converting the alkene analog of compound (2) into the epoxide of compound (3). Suitable epoxidizing agents include potassium peroxomonosulfate (oxone) in combination with acetone, *m*-CPBA, methyltrioxorhenium(VII), trifluoroper-acetic acid, and magnesium monoperoxyphthalate, with Oxone in combination with acetone, or m-CPBA being preferred. Possible solvents for the epoxidation of a compound of formula (2) include acetone, DMF, glyme, dioxane, CH₃CN, alcohols, THF, EtOAc, halohydrocarbons, chlorobenzene, dichloromethane and toluene. The reaction optionally takes place in the presence of a suitable base such as NaHCO₃. Reaction temperatures may range from about -30°C to about 50°C with about -10°C to about 25°C being preferred. The epoxide of formula (3) may be isolated and purified according to techniques and procedures well known in the art such as column chromatography. The α- and β- epoxides of formula (3) may be further separated by HPLC. It is preferred that the β-epoxide of formula (3), compound (3b), is separated from the α-epoxide of formula (3), compound (3a), and further used in the remaining steps of the process of this invention to form a the β-epoxy form of a compound of formula (I). However, the epoxidizing reaction of Scheme A, step 1 can also be used with the α-epoxide of formula (3a) or with a mixture of the two epoxides.

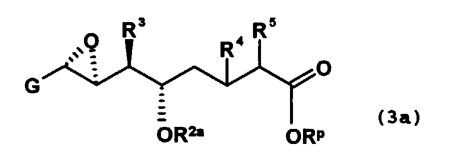

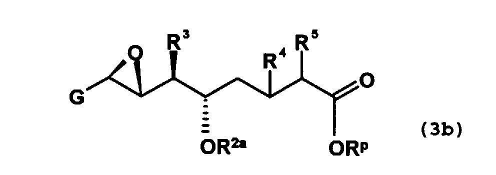

The compound of formula (2) wherein R^{p} is H may be epoxidized directly using *m*-CPBA. The *m*-CPBA epoxidation may be carried out on a compound of formula (2) to give a 1.2:1 β/α diastereomeric mixture of epoxides. The individual α-and β-diastereomers of (3) may be separated by HPLC, as described above. This direct epoxidation is illustrated in Scheme B. 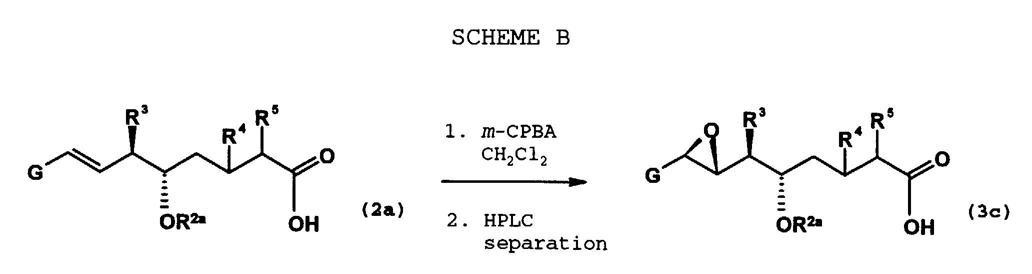 By eliminating the use of the N-hydroxysuccinimide ester, one step is eliminated from the synthesis.

Furthermore, a compound of formula (3c) may be prepared by deesterifying a compound (2b) according to Scheme B1. In Scheme B1, R^{a} is C₁-C₆ alkyl whereas all of the remaining substituents are as previously defined.

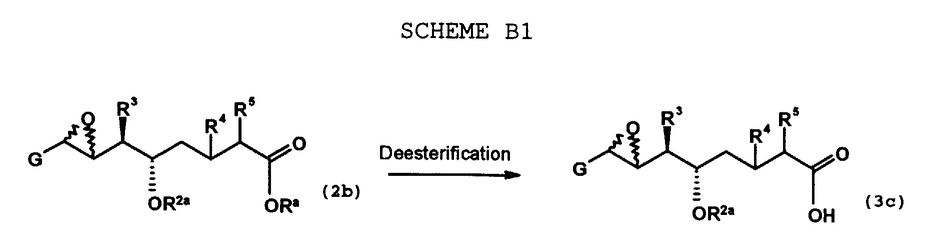

In Scheme B1, the alkyl ester of formula (2b) is deesterified with a suitable deesterifying agent to form the acid of formula (3c). The term "suitable deesterifying agent" encompasses any suitable means or conditions for removing the ester moiety of R^{a} while inert to the epoxide. For example, a suitable base, such as potassium hydroxide, is added to a solution of the alkyl ester of formula (2b) in a suitable solvent, such as tetrahydrofuran. The biphasic mixture is then allowed to stir at a temperature ranging from about 20°C to about 80°C, preferably 40°C and 65°C, for a period of from about 6 to 24 hours. After cooling to room temperature, the aqueous layer is washed with an appropriate acid, such as 1N hydrochloric acid, followed by brine. The mixture is dried, filtered and concentrated to provide the acid of (3c).

The ester of formula (2b) may be prepared by epoxidizing the alkene derivative of Preparation 1, step 10, which is also known in the art. Barrow, R.A. et al., *J. Am. Chem. Soc.* 117, 2479 (1995).

The compound of formula (2) may also be stereoselectively epoxidized to form either the compound of formula (3a) or (3b) using a chiral ketone with Oxone in the presence of a suitable base such as NaHCO₃ using procedures analogous to those disclosed by Tu, Y. et al, J. *Am. Chem. Soc*. 118, 9806 (1996); Wang, Z-X et al. *J. Org. Chem.* 62, 2328 (1997); Wang, Z-X et al., *J. Am. Chem. Soc*. 119, 11224 (1997). Preferred compounds of formula (2) for this reaction include those compounds where G is phenyl, R³ is methyl, R⁴ and R⁵ form a second bond and R is NHS. As used herein, the term "chiral ketone" refers to a ketone containing the following general features:
1) the stereogenic centers are close to the reacting center; and
2) the ketone has a fused ring and a a quaternary center a to a carbonyl group; and
3) one face of the ketone is sterically blocked. One especially preferred chiral ketone is of the structure:

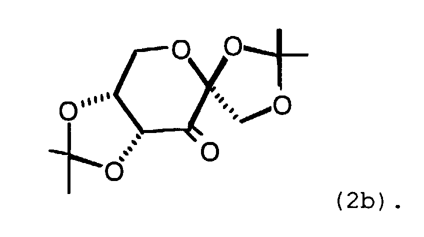

This preferred chiral ketone can be prepared from D-fructose by ketalization and oxidation under routine conditions. For example, the ketalization can be completed using acetone, HClO₄, and the process is conducted at about 0° C. For example, the oxidation can be completed using pyridinium chlorochromate at room temperature. These reactions are known in the art; see, for example: Tu, Y. et al, *supra.* and Wang, Z-X et al. *supra.* The asymmetric epoxidation can be carried out at a pH within the range of from about 7.0 to about 11.5 during the reaction.

Although it requires about 3-4 equivalents of chiral ketone to obtain conversions of greater than 95% with many cryptophycin intermediates at a pH of about 8.0, it is possible to use less chiral ketone (about 1-2 equivalents) at a pH of about 9.0 or above. Suitable solvents useful for the epoxidation step include H₂O, DMF, glyme, dioxane, CH₃CN, alcohols, THF, EtOAc, halohydrocarbons, chlorobenzene, and toluene, with a CH₃CN/H₂O solvent combination being preferred. Reaction temperatures may range from about -20°C to about 25°C with about -10°C to about 10°C being preferred. The individual isomers, (3a) or (3b), of crude product (3) can be isolated and purified by techniques well known in the art such as extraction, evaporation, chromatography and recrystallization. A preferred stereoselective epoxidation utilizes the chiral ketone of structure (2b) to provide a mixture of epoxides in the crude product (3) in the ratio of about α:β 1:5.

The compound of formula (2) where R^{p} is hydrogen is known or is readily prepared by methods known or analogously known in the art; PCT Intnl. Publ. No. WO 97/07798, published March 6, 1997; PCT Intnl. Publ. No. WO 96/40184, published December 19, 1996; Barrow, R.A. et al. *J. Am. Chem. Soc*. 117, 2479 (1995). The β-epoxide of formula (3) is generally preferred and is used throughout the process of this invention.

In Scheme A, step 2, the epoxide of formula (3) is coupled to the amino acid of formula (4) to yield a Fragment A-B compound of formula (5).

The amino acids of formula (4) are commercially available or are readily prepared by methods known in the art. Particularly preferred amino acids of formula (4) include those where R⁶ is a group of formula (IA) and R^{6a} is methoxy, R^{6b} is chloro and R^{6c} is H; R¹⁴ is hydrogen; and R^{p1} is hydrogen; said amino acids being disclosed by PCT Intnl. Publ. No. WO 97/07798, published March 6, 1997, PCT Intnl. Publ. No. WO 96/40184, published December 19, 1996; Barrow, R.A. et al. *J. Am. Chem. Soc*. 117, 2479 (1995).

The epoxide of formula (3), where R^{p} is NHS, is coupled to the amino acid of formula (4) according to coupling procedures which are inert to the epoxide functionality. For example, the epoxide of formula (3) is contacted with from about 1.5 to 3.5 equivalents of amino acid (4), where R^{p1} and R¹⁴ are both hydrogen, and a suitable silylating agent in the presence of a suitable organic solvent. Suitable organic solvents include DMF, glyme, dioxane, CH3CN, THF, EtOAc, and halohydrocarbons, such as methylene chloride. The reaction is carried out at a temperature ranging from about -30°C to about 75°C, with a temperature ranging from about 20°C to about 60°C being preferred. The fragment A-B compound of formula (5) may be isolated and purified according to techniques and procedures well known in the art such as extraction, evaporation, chromatography and recrystallization.

As used herein, the term "silylating agent" is selected from any reagent capable of attaching a silyl group to a target substituent. Generally known silylating agents are employed. See for example, Calvin, E.W., "Silicon Reagents in Organic Synthesis", Academic Press, (London, 1988). Generally typical silyl agents include any reagent with a trialkylsilyl group such as trimethylsilyl, triethylsilyl, triisopropylsilyl, dimethylisopropylsilyl, diethylisopropylsilyl, dimethylthexylsilyl, and t-butyldimethylsilyl, any reagent with an alkylarylsilyl group such as tribenzylsilyl, diphenylmethylsilyl, t-butylmethoxyphenylsilyl and tri-p-xylylsilyl, and any reagent with a triarylsilyl group such as triphenylsilyl. The preferred silylating agent is a trimethyl silylating agent. Typical trimethyl silylating agents include N,O-Bis(trimethyl silyl) acetamide, allyltrimethylsilane, N,O-Bis(trimethylsilyl)- carbamate N,N-Bis(trimethylsilyl)methylamine, Bis(trimethylsilyl)sulfate, N,O-Bis(trimethylsilyl)trifluoroacetamide, N,N-Bis(trimethylsilyl)urea, (ethylthio)trimethylsilane, ethyl trimethyl silylacetate, hexamethyldisilane, hexamethyldisilazane, hexamethyldisiloxane, hexamethyldisilthiane, (isopropenyloxy) trimethyl silane, 1-methoxy-2-methyl-1-trimethyl-siloxy-propene, (methylthio)trimethlysilane, methyl 3-trimethylsiloxy-2-butenoate, N-methyl-N-trimethylsilylacetamide, methyl trimethylsilylacetate, N-methyl-N-trimethylsilyl-heptafluorobutyramide, N-methyl-N-trimethylsilyl-trifluoroacetamide, (phenylthio)trimethylsilane, trimethylbromosilane, trimethylchlorosilane, trimethyliodosilane, 4-trimethylsiloxy-3-penten-2-one, N-(trimethylsilyl)acetamide, trimethylsilyl acetate, trimethylsilyl azide, trimethylsilyl benzenesulfonate, trimethylsilyl cyanide, N-trimethylsilyldiethylamine, N-trimethylsilyldimethylamine, trimethylsilyl N,N-dimethylcarbamate, 1-(trimethylsilyl)imidazole, trimethylsilyl methanesulfonate, 4-(trimethylsilyl)morpholine, 3-trimethylsilyl-2-oxazolidinone, trimethylsilyl trichloroacetate, trimethylsilyl trifluoroacetate and trimethylsilyl trifluoromethane sulfonate. Particularly useful silylating agents include "tri-lower alkyl silyl" agents, the term of which contemplates triisopropylsilyl, trimethylsilyl and triethylsilyl, trimethylsilyl halides, silylated ureas such as bis(trimethylsilyl)urea (BSU) and silylated amides such as N,O-bis(trimethylsilyl)acetamide (BSA). Bis N,O-trimethyl silyl acetamide (BSA) is an especially preferred silylating agent.

Alternatively, the desired β-epoxide (3c) may be coupled with (4), when R^{p1} is hydrogen, using a suitable coupling agent, preferably diphenylphosphinic chloride, and a silyl agent to give fragment A-B (5). Suitable coupling agents are well known in the art, as described by Greene, T.W. "Protecting Groups in Organic Synthesis", Wiley (New York, 1981) and include N,O- diphenylphosphinic chloride, diphenyl chlorophosphate, DCC, EDCI, chloroformates, and 2-chloro-4,6-dimethoxy-1,3,5-triazine. Dipehnylphosphinic chloride is a preferred coupling agent. The suitable organic solvents described above, preferably methylene chloride, may be used. This procedure allows for the elimination of the carboxy protection step and allows for the use of lower amounts of amino acid (4).

In Scheme A, step 3, the fragment A-B compound of formula (5) is deprotected with a suitable alkoxy deprotecting agent to form a compound of formula (6).

A suitable alkoxy deprotecting agent is one that removes the hydroxy protecting group signified by the R^{2a} substituent while inert to the epoxide moiety of the fragment A-B compound of formula (5). Preferred deprotecting agents include basic fluoride sources such as tetrabutylammonium fluoride, pyridinium fluoride, triethylammonium fluoride, cesium fluoride, and the like, with tetrabutylammonium fluoride being preferred. The deprotection reaction takes place in the presence of a suitable organic solvent such as tetrahydrofuran, optionally in the presence of a suitable base, such as sodium bicarbonate (NaHCO₃). The reaction takes place in the range of from about 0°C to about 80°C with from about 20°C to about 70°C being preferred. The reaction is run for a period of time ranging from about 3 to 24 hours. Crude product (6) may be used without further purification. Alternatively, the compound of formula (6) may be isolated and purified according to procedures well known well known in the art such as extraction, evaporation, chromatography and recrystallization.

When R^{p1} for the compound of formula (6) is hydrogen, the R^{p1} moiety is actually the cationic salt of deprotecting agent, for example, cesium, tetrabutylammonium, and the like.

In Scheme A, step 4, the compound of formula (6) is contacted with a thioester forming agent to provide the ester of formula (7).

The term "thioester forming agent" encompasses any suitable means or conditions for forming the thioester moiety of formula (7). Included within this definition are the conditions set forth and/or analogously described in Ono, N. et al., *Bull. Chem. Soc*. *Jpn.* 51 (8), 2401 (1978); Ho, Tse-Lok, *Synth. Comm.* 9(4), 267-270 (1979); Narasaka, K. et al., *J. Am. Chem. Soc.* 106 (10), 2954-2960 (1984); L.G. Wade, Jr. et al., *Tetrahedron Lett.* 731-732 (1978); Mora, N. et al., *Tetrahedron Lett.* 34 (15), 2461-2464 (1993); and Dossena, A. et al. *J. Chem. Soc. Perkin Trans. I,* 2737 (1981).

For example, the compound of formula (6) may be treated with a sterically hindered alkyl halide, such as tert-butylbromide, and a solvent of the formula (R⁸¹) (Me)SO, wherein R⁸¹ is as defined above, in the presence of a suitable base, such as sodium bicarbonate (NaHCO₃). A preferred solvent for reaction is dimethylsulfoxide (DMSO). Both the sterically hindered alkyl halide and the suitable base are added in a molar excess of about 7.0 to 12.0 in comparison to the compound of formula (6). The reaction takes place in the range of from about 0°C to about 60°C with from about 10°C to about 30°C being preferred. The reaction is run for a period of time ranging from about 1 to 24 hours. Crude product (7) may be used without further purification. Alternatively, the ester of formula (7) may be isolated and purified according to procedures well known well known in the art such as extraction, evaporation, chromatography and recrystallization.

In those instances when the substituent R^{p1} is a moiety other than hydrogen, the compound of formula (6) must first be carboxy-deprotected. Carboxy-deprotections under basic conditions are known by those of ordinary skill in the art. For example, the compound of formula (6) may be treated with a suitable base, such as lithium hydroxide (LiOH) for a period of time sufficient to remove the carboxy protecting group, for example from about 1 to 24 hours.

In Scheme A, step 5, the ester of formula (7) is coupled with a carboxylic acid of formula (8) to provide the compound of formula (9).

For example, the carboxylic acid of formula (8) is dissolved in a suitable organic solvent, such as DMF, glyme, dioxane, THF, CH₃CN, EtOAc, and halohydrocarbons, with dichloromethane being preferred. This solution is then treated with a coupling reagent. Possible coupling reagents include DCC, EDCI, and similar reagents, such as DMAP which activate carboxylic acids towards esterification with alcohols. This solution may then be optionally treated with a suitable base such as solid sodium bicarbonate and then contacted with an ester of formula (7). The concentration of (8) after these additions should range from about 0.1 M to about 2.0 M. The reaction takes place in the range of from about -30°C to about 60°C with from about 10°C to about 30°C being preferred. The reaction is run for a period of time ranging from about 0.5 to 12 hours. The final concentration of Crude product (9) may be used without further purification. Alternatively, the compound of formula (9) may be isolated and purified according to procedures well known well known in the art such as extraction, evaporation, chromatography and recrystallization.

In Scheme A, step 6, the compound of formula (9) is oxidized with a suitable oxidizing agent to provide the sulfone or sulfoxide of formula (10).

A suitable oxidizing agent is an agent capable of converting the sulfide of formula (9) into the sulfone of formula (10), while inert to the epoxide moiety of the molecule. Suitable oxidizing agents include potassium peroxomonosulfate (Oxone), *m*-CPBA, methyltrioxorhenium(VII), and magnesium monoperoxyphthalate, with Oxone being preferred.

For example, the sulfide of formula (9) is treated with a suitable base, such as sodium bicarbonate followed by a suitable oxidizing agent, such as Oxone. The reaction is carried out in a suitable solvent, such as acetone, DMF, glyme, dioxane, CH₃CN, alcohols, THF, EtOAc, halohydrocarbons, chlorobenzene, and toluene, with acetone being preferred. Generally, the reaction is carried out at temperatures of from about -30°C to about 50°C with from about -10°C to about 10°C being preferred. Generally, the reaction requires from about 15 minutes to about 5 hours. Crude sulfone or sulfoxide (10) may be used without further purification. Alternatively, the sulfone or sulfoxide of formula (10) may be isolated and purified according to procedures well known well known in the art such as extraction, evaporation, chromatography and recrystallization.

In Scheme A, step 7, the sulfone or sulfoxide of formula (10) is deprotected with a suitable deprotecting agent to provide the amine of formula (10a).

A suitable deprotecting agent is an agent capable of removing the base labile substituent R⁸² on the compound of formula (10) while inert to the epoxide moiety of the molecule. Suitable deprotecting agents include bases such as secondary and tertiary amines and inorganic bases, for example, piperidine, morpholine, dicyclohexylamine, *p*-dimethylaminopyridine, diisopropylethylamine, and the like, with piperidine being preferred. The reaction is carried out in a suitable solvent such as DMF, glyme, dioxane, CH₃CN, alcohols, THF, EtOAc, halohydrocarbons, chlorobenzene, or toluene. Generally, the reaction is carried out at a temperature ranging from about 0°C to about 120 °C. Generally, the reaction requires from about 1 to 72 hours. The compound of formula (I) may be isolated and purified by techniques well known in the art, such as extraction, evaporation, chromatography and recrystallization. Alternatively, the compound of formula (10a) is isolated and may be further cyclized with a cyclizing agent to provide a compound of formula (I).

Typically, once the compound of formula (10) is deprotected, it undergoes spontaneous cyclization. However, some particular compounds of formula (10) may require an additional cyclization step. Also, for example, the sulfide of formula (9), although much less active than its oxidized counterpart, upon removal of the base-labile protecting group may be cyclized with a suitable cyclizing agent, such as 2-hydroxypyridine to form a compound of formula (I). For example, the sulfide of formula (9), or alternatively a selected compound of formula (10a), is heated in a suitable solvent, such as DMF at about 60 °C for several days in the presence of piperidine and 2-hydroxypyridine. The compound of formula (I) is isolated and purified by techniques well known in the art, such as extraction, evaporation, chromatography and recrystallization.

Optionally, on those compounds of formula (I) containing basic or acidic functional groups, pharmaceutically acceptable salts of the compounds of formula (I) may be formed using standard techniques. For example, the free base may be dissolved in aqueous or aqueous-alcohol solution or other suitable solvent containing the appropriate acid and the salt isolated by evaporating the solution. Alternatively, the free base may be reacted in an organic solvent containing the appropriate acid and the salt isolated by evaporating the solution. Further, the free base may be reacted in an organic solvent in which case the salt separates directly or can be obtained by concentration of the solution or in a solvent such as water which is then removed in vacuo or by freeze-drying, or by exchanging the cations of an existing salt for another cation on a suitable ion exchange resin.

A synthetic scheme for making the carboxylic acids of formula (8) is set forth in Scheme C. The reagents and starting material are readily available to one of ordinary skill in the art. In Scheme C, all substituents, unless otherwise indicated, are as previously defined.

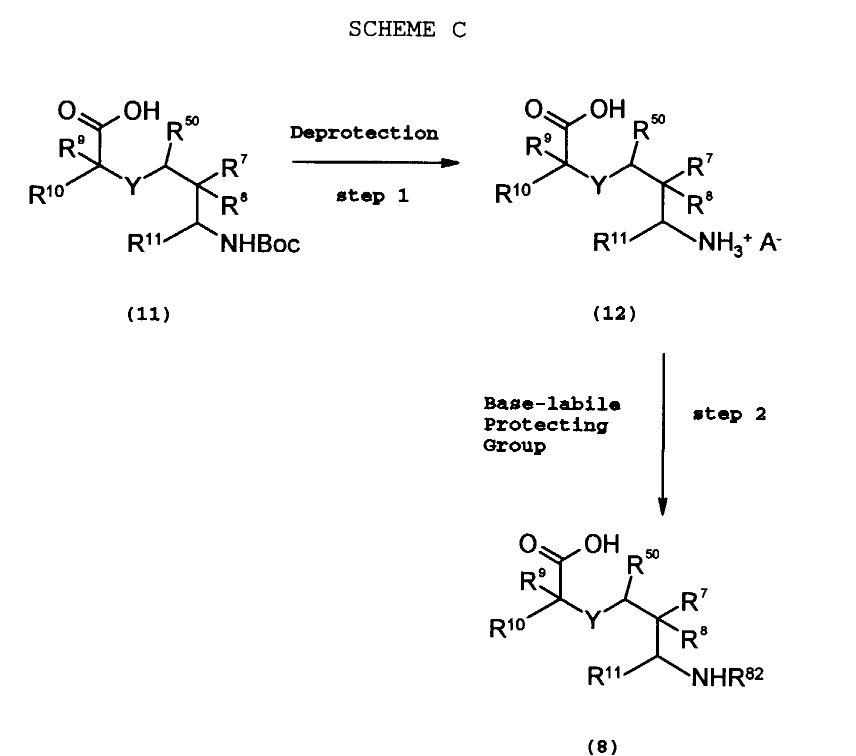

In Scheme C, step 1, the Boc-protected amine of formula (11) is deprotected to provide the deprotected amine of formula (12).

For example, the deprotection reaction involves the removal of an amino protecting group by techniques and procedures well known and appreciated by one of ordinary skill in the art. The selection, use, and removal of protecting groups are set forth by Greene, T.W. "Protecting Groups in Organic Synthesis", Wiley (New York, 1981). For example, the Boc-protected amine of formula (11) is dissolved in a suitable acid, such as trifluoroacetic acid or hyrdrochloric acid. Generally, the reaction is carried out at a temperature ranging from about 0°C to about 60 °C. Generally, the reaction requires from about 1 to 24 hours. The deprotected amine of formula (12) may be isolated and purified by techniques well known in the art, such as extraction, evaporation, chromatography and recrystallization.

The Boc-protected amine of formula (11) is described in Barrow, R.A. et al. J. *Am. Chem. Soc.* 117, 2479 (1995); PCT Intnl. Publ. No. WO 96/40184, published December 19, 1996; and PCT Intnl. Publ. No. WO 97/07798, published March 6, 1997.

In Scheme C, step 2, the deprotected amine of formula (12) is amino-protected with a base-labile amino protecting group to provide the carboxylic acid of formula (8).

For example, the protection of an amino group with a base-labile amino protecting group involves the addition of a base-labile amino protecting group by techniques and procedures well known and appreciated by one of ordinary skill in the art. The selection, use, and removal of base-labile amino protecting groups are set forth by Greene, T.W. "Protecting Groups in Organic Synthesis", Wiley (New York, 1981). A preferred base-labile amino protecting group is Fmoc. For example, to a solution of the deprotected amine of formula (12) in a suitable solvent, such as dioxane, is added a suitable base, such as sodium bicarbonate, followed by a compound of the formula R⁸²-Cl or R⁸²-ONHS, such as Fmoc-Cl or Fmoc-ONHS succinimide. The mixture may be optionally diluted with a small amount of water and stirred for a period of time ranging from 12 to 48 hours at a temperature ranging from about 0°C to about 60°C. The mixture may be quenched with a suitable acid, such as hydrochloric acid. The carboxylic acid of formula (8) may be isolated and purified by techniques well known in the art, such as extraction, evaporation, chromatography and recrystallization.

The pharmaceutically acceptable salts of a compound of formula (I) may optionally be formed according to the procedures described in Scheme A.

Some preferred characteristics of this invention are set forth in the following tabular form wherein the features may be independently selected to provide preferred embodiments of this invention. The invention is in no way limited to the features described below:
A) R⁸ is ethyl, propyl, isopropyl, butyl, isobutyl or isopentyl;
B) R⁷ is ethyl, propyl, isopropyl, butyl, isobutyl, pentyl, or isopentyl;
C) R⁷ is H, R⁸ is methyl, R³ is methyl;
D) R³ is ethyl, propyl, isopropyl, butyl, isobutyl, pentyl or isopentyl;
E) R⁹ is methyl, ethyl, propyl, butyl, isobutyl, pentyl, or isopentyl;
F) R¹⁰ is methyl, ethyl, propyl, butyl, isobutyl, pentyl, or isopentyl;
G) Ar is phenyl optionally substituted with a substituent selected from the group consisting of hydrogen, halogen, -CH₂OC(O) (CH₂)ₘ,NH₂ and simple alkyl;
H) a compound wherein Y is selected from the group consisting of O, NH, S, SO and SO₂;
I) a compound wherein Y is CH, and R⁷, R⁸, R⁹, and R¹⁰ are each hydrogen;
J) R⁷, R⁸ are each hydrogen;
K) R⁷ and R⁸ are each selected from hydrogen or OH;
L) R⁷ and R⁸ are each methyl;
M) R⁷ and R⁸ together form a cyclopropyl group;
N) Y is NH;
O) R⁴ and R⁵ form a double bond;
P) R⁶ is substituted benzyl wherein one substituent is a halogen and one is an OR¹² group wherein R¹² is lower alkyl;
Q) the epoxidizing agent is Oxone;
R) the epoxidation is selective;
S) the chiral ketone is of the formula: 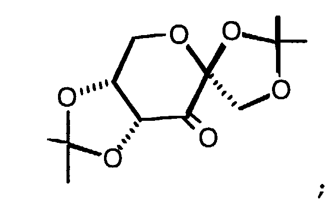 and
T) R^{p} is NHS or hydrogen.

The ester starting material can be prepared, for example, according to Scheme D. The reagents and starting material are readily available to one of ordinary skill in the art. In Scheme D, all substituents, unless otherwise indicated, are as previously defined.

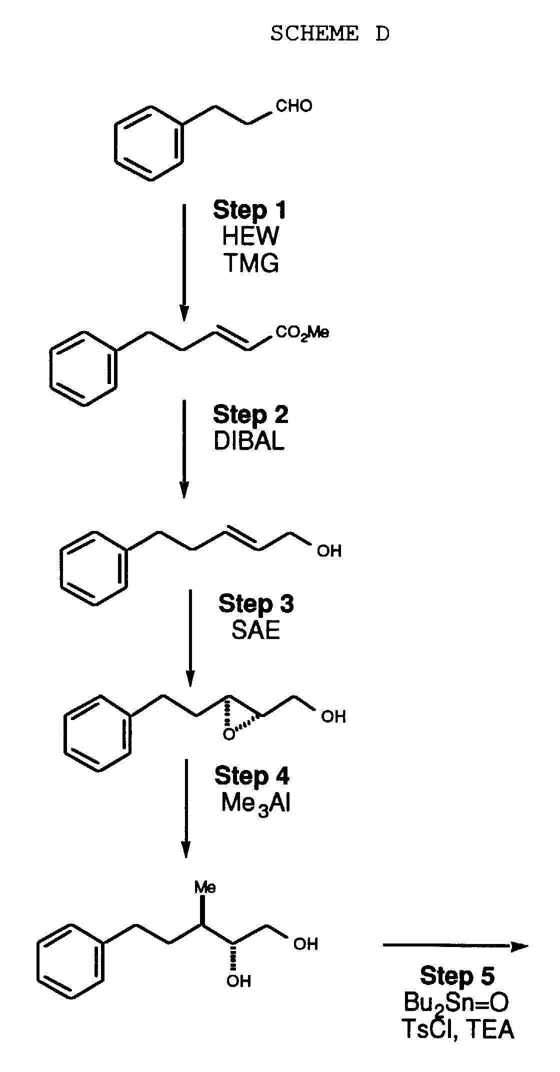

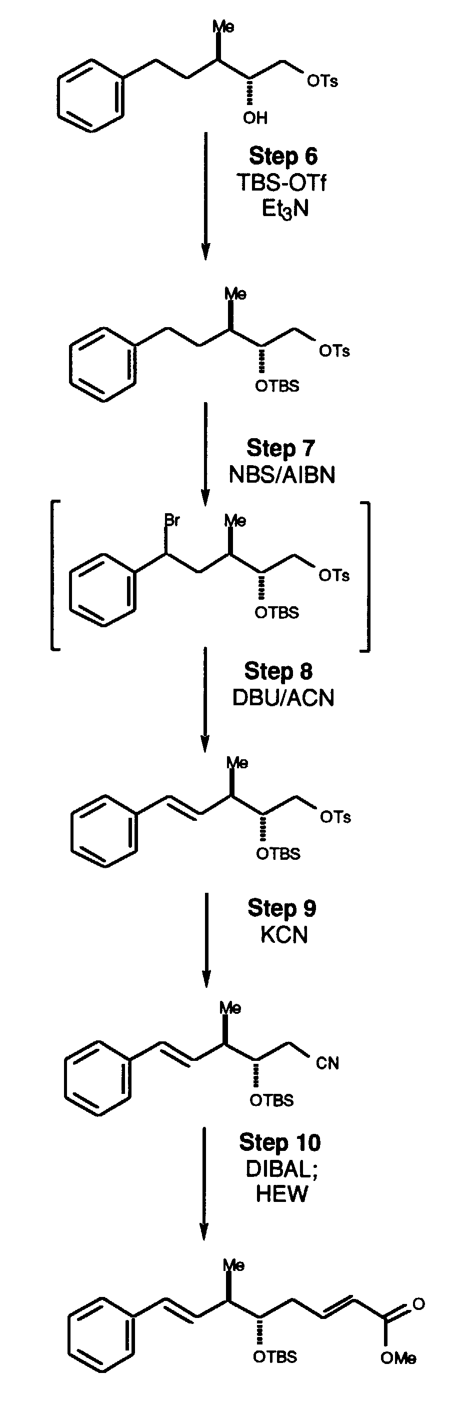

The scheme for preparing the ester is further explained by the Preparation Section herein which provides one specific application of the scheme for the convenience of the skilled artisan.

Scheme D for preparing the ester is applicable to the Ar substituents claimed herein. The scheme illustration is not intended to limited the synthesis scheme only to the phenyl ring illustrated. Rather, the artisan can broadly apply this process to provide desired starting materials for use in the processes claimed herein.

The necessary reaction time is related to the starting materials and operating temperature. The optimum reaction time for a given process is, as always, a compromise which is determined by considering the competing goals of throughput, which is favored by short reaction times, and maximum yield, which is favored by long reaction times.

To further illustrate the invention the following examples are provided. The scope of the invention is in no way to be construed as limited to or by the following examples.

### Preparation 1

### Step 1. Methyl 5-Phenylpent-2(E)-enoate.

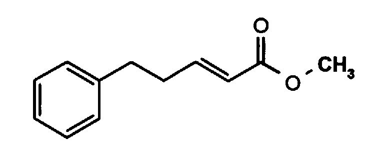

A solution of trimethyl phosphonoacetate (376 g, 417 mL, 2.07 mol) in THF (750 mL) was stirred at 0°C in a 3L 3-neck round bottom flask equipped with a mechanical stirrer and N₂ inlet. To the chilled solution, neat tetramethyl guanidine (239 g, 260 mL, 2.07 mol) was added dropwise via an addition funnel. The chilled clear pale yellow solution was stirred for 25 minutes at 0 °C. A solution of hydrocinnamaldehyde (90%, 253 g, 248 mL, 1.9 mol) in THF (125 mL) was added dropwise to the reaction solution slowly. Upon completion of addition, the reaction was stirred for 10 h rising to room temperature. GC indicated a 95:5 ratio of product to starting material. 500ml of water was added to the reaction vessel and the reaction stirred overnight separating into two layers. The organic layer was isolated and the aqueous layer was extracted with t-BuOMe. The organic layers were combined and dried over MgSO₄, then concentrated *in vacuo* to yield an orange oil. The crude product was distilled at 129 °C/0.3mm Hg yielding 360.5g, 91.7% yield, of a clear slightly yellow oil.
EIMS *m/z* 190(13; M+), 159(410, 158(39), 131(90), 130(62), 117(22), 104(12), 95(57), 91(100), 77(21), 65(59); HREIMS *m/z* 190.0998 (C₁₂H₁₄O₂ **D** -0.4 mnu); UV lmax (e) 210 (8400), 260 (230) nm; IR nmax 3027, 2949, 1723, 1658, 1454, 1319, 1203, 978, 700 cm⁻¹; ¹H NMR d (CDCl₃) 7.15-7.3 (Ph-H5;bm), 7.00 (3-H;dt, 15.6/6.6), 5.84 (2-H;dt, 15.6/1.2), 3.70 (OMe;s), 2.76 (5-H2;t, 7.2), 2.51 (4-H2; bdt, 6.6/7.2); ¹³C NMR d (CDCl₃) 166.9 (1), 148.3(3), 140.6(Ph-1'), 128.4/128.2 (Ph2'/3'/5'6'), 126.1 (Ph 4'), 121.4 (2). 51.3 (OMe), 34.2/33.8 (4/5).

### Step 2. 5-phenyl-pent-2-en-1-ol.

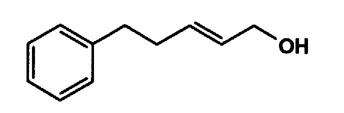

To a 12L 4-neck round bottom flask equipped with a thermocouple, mechanical stirrer and N₂ inlet, a solution of enoate ester of Preparation 1, step 1 (310.5 g, 1.5 mol) in THF (1.5 L) was charged and chilled to -71 °C via a *i*-PrOH/CO₂ bath. To the reaction vessel, was added dropwise DIBAL (2.5 L, 1.5 M in toluene, 3.75 mol) at a rate to maintain the reaction temperature < -50 °C. Upon complete addition, the reaction was stirred overnight with the reaction temperature < -50 °C. TLC (3:1 Hexanes:EtOAc, SiO₂) indicated absence of starting material after 16 h. The reaction temperature was allowed to raise to -15°C. The reaction was quenched slowly withlN HCl (150 mL). At this point the reaction setup into a gelatinous solid. A spatula was employed to breakup the the semi-solid and 1N HCl (200 mL) was added making the mixture more fluid. Concentrated HCl (625 mL) was charged to form a two phase system. The layers were separated and the product extracted with t-BuOMe. The organic layer was dried over MgSO₄ and concentrated *in vacuo* to yield a clear pale yellow oil, 247.8g. The crude product was distilled at 145 °C/0.25mm Hg yielding 209.7g, 86.2%.
EIMS *m/z* 162 (1:M+) 144 (16), 129 (7), 117 (9) 108 (6), 92 (17), 91 (100), 75 (5), 65 (12), HREIMS *m/z* 162, 1049 (C₁₁H₁₄O, D -0.4 mmu); UV lmax (e) 206 (9900), 260 (360); IR nmax 3356, 2924, 1603, 1496, 1454, 970, 746, 700 cm⁻¹; ¹H NMR d 7.15-7.3 (Ph-H5;m), 5.70 (3-H;dt, 15.6/6.0), 5.61 (2-H;dt, 15.6/4.8), 4.02 (1-H2;d 4.8), 2.68 (5-H2; t, 7.2), 2.40 (OH;bs), 2.36(4-H2; dt, 6.0/7.2); ¹³C NMR d141.6 (Ph 1'), 131.8(3), 129.5 (2), 128.3/128.2 (Ph 2'/3'/5'/6'), 125.7 (Ph 4'), 63.3 (1), 35.4/33.8 (4/5).

### Step 3. (2S,3S)-2,3-Epoxy-5-phenyl-1-pentanol.

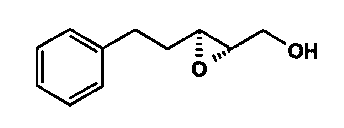

To a 1L 3 neck round bottom flask equipped with a mechanical stirrer, thermocouple and nitrogen inlet was added CH₂Cl₂ (350 mL), dried 4 Å molecular sieves (30 g) and L-(+)-diethyl tartrate (7.62 g, 0.037 mol). The resulting mixture was cooled to -20 °C and treated with Ti(O-*i*-Pr)₄ (9.2 mL, 0.031 mol), followed by the addition of *t*-butylhydroperoxide (4.0 M in CH₂Cl₂, 182 mL, 0.78 mol) at a rate to maintain the temperature ² -20 °C. Upon complete addition, the reaction mixture was stirred for another 30 min, and then treated with a solution of the allylic alcohol of Preparation 1, step 2 (50 g, 0.31 mol) in CH₂Cl₂ (30 mL) at a rate to maintain the temperature ² -20 °C. The reaction was stirred at the same temperature for 5 h, then filtered into a solution of ferrous sulfate heptahydrate (132 g) and tartaric acid (40 g) in water (400 mL) at 0 °C. The mixture was stirred for 20 min, then transferred to a separatory funnel and extracted with *t*-BuOMe (2x200 mL). The combined organic phase was stirred with 30% NaOH solution containing NaCl, for 1 h at 0 °C. The layers were again separated, and the aqueous phase extracted with *t*-BuOMe. The combined organic phase was washed with brine, dried over MgSO₄ and concentrated to yield 52.8 g as an amber oil.

### Step 4. (2R, 3R)-2-hydroxy-3-methyl-5-phenylpentan-1-ol .

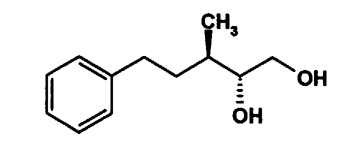

To a 5L 3 neck round bottom flask equipped with a mechanical stirrer, thermocouple and nitrogen inlet was added hexanes (1L) and cooled to 0 °C. A 2.0M solution of Me₃Al in hexanes (800 mL, 1.6 mol) was added, followed by a solution of the epoxide of Preparation 1, step 3 (120 g, 0.677 mol) in hexanes (250 mL)/CH₂Cl₂ (50 mL) maintaining the temperature below 20 °C. Upon complete addition, the cloudy reaction mixture was stirred at 5 °C for 35 min, whereupon a solution of 10% HCl (300 mL) was added dropwise, followed by the addition of concd HCl (350 mL). The layers were separated, and the organic phase was washed with brine and dried over MgSO₄. After removal of the volatiles *in vacuo,* 122.1 gram of an oil was obtained.

### Step 5. (2R, 3R)-2-hydroxy-3-methyl-5-phenylpent-1-yl Tosylate.

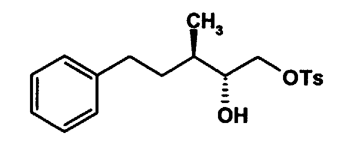

To a 2L 3 neck round bottom flask equipped with a mechanical stirrer and nitrogen inlet was added the diol of Preparation 1, step 4 (58 g, 0.30 mol), dibutyltin oxide (1.5 g, 0.006 mol, 2 mol%), toluenesulfonyl chloride (57.5 g, 0.30 mol), CH₂Cl₂ (580 mL) and triethylamine (42.0 mL, 0.30 mol). The resulting mixture was stirred at room temperature for 2 h (although the reaction was complete within 1 h), filtered, washed with water and dried over MgSO₄. Concentration of the volatiles *in vacuo* afforded 104.1 gram of a slightly amber oil.

### Step 6. (2R, 3R)-2-[(tert-Butyldimethylsilyl)oxy]-3-methyl-5-phenylpent-1-yl Tosylate.

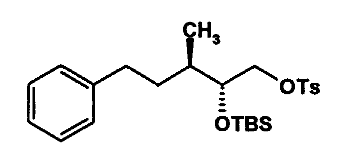

A solution of the tosylate of Preparation 1, step 5 (100 g, 0.29 mol) and triethylamine (81.0 mL, 0.58 mol) in CH₂Cl₂ (1200 mL) was treated with neat TBS-OTf (99 mL, 0.43 mol) dropwise with continued stirring for another 20 min. The reaction was washed twice with brine, dried over MgSO₄ and concentrated to dryness. The oil was dissolved in a minimal amount of hexanes and filtered over a silica pad, eluting with hexanes:EtOAc (9:1) to yield a slightly amber oil, 134 g.

### Step 7. (2R, 3R,5RS)-2-[(tert-Butyldimethylsilyl)oxy]-3-methyl-5-bromo-5-phenylpent-1-yl Tosylate.

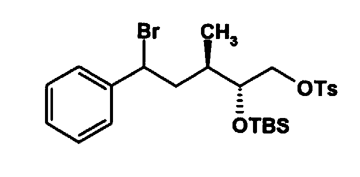

To a 5L 3 neck round bottom flask equipped with a mechanical stirrer, reflux condenser and nitrogen inlet was added CCl₄ (1680 mL), TBS Ts product of Preparation 1, step 6 (140 g, 0.30 mol), NBS (65g, 0.365 mol) and AIBN (16.5 g, 0.10 mol). The mixture was degassed by evacuation under full vacuum with stirring, and backfilling with nitrogen (3x). The reaction mixture was then heated to reflux, whereupon the color became dark brown. After 15 min at vigorous reflux, the reaction mixture became light yellow, and chromatographic analysis indicated the reaction was complete. After cooling to room temperature, the reaction was filtered and the filtrate concentrated to dryness. The residue was redissolved in hexanes and filtered again, and concentrated to dryness to afford 170.3 gram as an amber oil.

### Step 8. (2R, 3R)-2-[(tert-Butyldimethylsilyl)oxy]-3-methyl-5-phenylpent-4(E)-en-1-yl Tosylate.

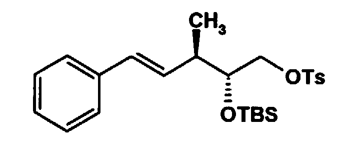

To a 2L 3 neck round bottom flask equipped with a mechanical stirrer, reflux condenser and nitrogen inlet was added a solution of the bromide of Preparation 1, step 7 (100 g, 0.186 mol) in acetonitrile (700 mL). DBU (83.6 mL, 0.557 mol) was added and the resulting dark brown solution was stirred at reflux for 15 min. After cooling to room temperature, the solvent was removed *in vacuo,* and the residue digested in CH₂Cl₂ (200 mL) and filtered through a silica pad. The volatiles were again evaporated, and the residue dissolved in EtOAc and washed with water, brine and dried over MgSO₄ and concentrated to dryness. Preparative mplc (Prep 500) chromatography afforded the desired unsaturated compound (50.3 g, 60% yield over 4 steps).

### Step 9. (3S, 4R)-3-[(tert-Butyldimethylsilyl)oxy]-4-methyl-6-phenylhex-5(E)-en-1-nitrile.

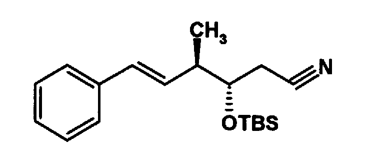

The tosylate of Preparation 1, step 8 (50 g, 0.11 mol) was dissolved in DMSO (1 L) and treated with KCN (14.2 g, 0.22 mol) and water (25 mL), and the resulting mixture was stirred at 60 °C under nitrogen for 18 h. After cooling to room temperature, the reaction mixture was partitioned between EtOAc (1 L) and water (1 L). The aqueous phase was extracted with EtOAc (500 mL), and the combined organic phase was washed with brine and dried over Na₂SO₄. Flash chromatography over silica with CH₂Cl₂ afforded the desired nitrile in 92% yield.

### Step 10. Methyl (5S, 6R)-5-[(tert-Butyldimethylsilyl)oxy]-6-methyl-8-phenylocta-2(E),7(E)-dienoate.

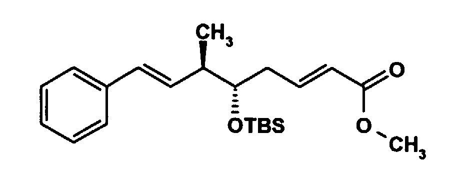

The nitrile of Preparation 1, step 9 (14.67 g, 46.5 mmol) was dissolved in toluene (200 mL) and cooled to -78 °C under nitrogen. A 1.5M solution of DIBAL in toluene (37.2 mL, 55.8 mmol) was added dropwise with vigorous stirring. Upon complete addition, the cooling bath was removed and the reaction was stirred at room temperature for 1 h. The reaction mixture was carefully poured into 1N HCl and the mixture stirred at room temperature for 30 min. The layers were separated, and the organic phase was washed with a saturated aqueous solution of sodium potassium tartrate (2x), brine and dried over Na₂SO₄. The volatiles were removed *in vacuo,* and the crude pale yellow oil was used directly in the subsequent condensation.
The crude aldehyde from above was dissolved in THF (90 mL) and treated with trimethyl phosphonoacetate (9.03 mL, 55.8 mmol) and tetramethylguanidine (7.0 mL, 55.8 mmol) at room temperature under nitrogen. The reaction mixture was stirred for 16 h, then partitioned between EtOAc (200 mL) and water (100 mL). The aqueous phase was back extracted with EtOAc (100 mL), and the combined organic phase was washed with water, brine and dried over Na₂SO₄. The volatiles were removed *in vacuo,* and the crude yellow oil (17.0 g) was chromatographed over silica gel with CH₂Cl₂ : cyclohexane (1 : 1 to 2 : 1) to afford 13.67 grams of the desired ester, 78.5%.

### Preparation 2

### (5S,6R)-5-[(tert-Butyldimethylsilyl)oxy]-6-methyl-8-phenylocta-2(E),7(E)-dienoic acid

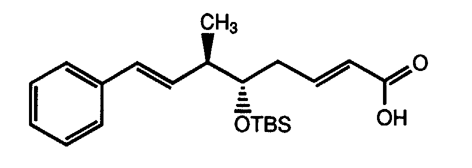

Methyl (5*S*, 6*R*)-5-[(*tert*-Butyldimethylsilyl)oxy]-6-methyl-8-phenylocta-2(*E*),7(*E*)-dienoate from Preparation 1, step 10 (1.00g, 2.673 mmol) was dissolved in acetone (44mL) and then 1N aqueous LiOH (26mL) added at room temperature. The cloudy mixture was further diluted with acetone (20mL) and the resulting yellow mixture stirred at room temperature for 23.5h. The reaction was diluted with diethylether (400mL) and the organics washed with 1N Hcl (120mL), brine (200mL) and H₂O (160mL). The organics were dried (MgSO₄) and concentrated *in vacuo* to leave a yellow oil which was purified by column chromatography (gradient elution: 5% AcOH + 20%-40% EtOAc/Hexanes) to give carboxylic acid as a yellow oil (960mg, 100%).
[α]_{D}⁵⁸⁹ +87.6° (c 10.5, CHCl₃); ¹H NMR (CDCl₃) d 7.38-7.19 (m, PhH₅), 7.09 (ddd, J = 15.2, 7.6 and 7.9 Hz, 3-H), 6.38 (d, J = 16 Hz, 8-H), 6.16 (dd, J = 16 and 8 Hz, 7-H), 5.85 (d, J = 15.8 Hz, 2-H), 3.81-3.75 (m, 5-H), 2.49-2.37 (m, 6-H, 4-HH'), 1.12 (d, J = 6.7 Hz, 6-Me), 0.91 (s, 9H, SiCMe₃), 0.065 (s, SiMe), 0.068 (s, SiMe) ppm; IR (CHCl₃) lₘₐₓ 2957, 2930, 2858, 1697, 1258, 1098, 838 cm⁻¹; MS (FD) 360.2 (M⁺,100); Anal. calcd. for C₂₁H₃₂O₃ requires: C, 69.95; H,8.95%. Found: C,69.19; H,8.39%.

### Preparation 3

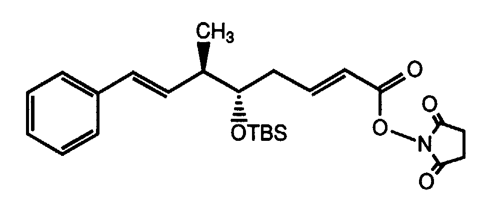

To a stirred solution of the carboxylic acid of Preparation 2 (720mg, 2 mmol) in dry dimethylformamide (5.50mL) was added 1-Ethyl-3-(3-dimethyaminopropyl)carbodiimide (459mg, 2.4 mmol) and N-hydroxysuccinimide (299mg, 2.6mmol) at room temperature. The mixture was stirred for 28h and then diluted with EtOAc (100mL) and washed with 1N aqueous HCl (2x50mL), H₂O (75mL), dried (MgSO4) and concentrated in vacuo to leave an oil. Crude product was purified by column chromatography (gradient elution : 5-30% EtOAc/Hexanes) to give active ester as a pale yellow oil (724mg,80%).
[α]_{D}⁵⁸⁹ +71.3° (c 10.1, CHCl₃); ¹H NMR (CDCl₃) δ 7.36-7.20 (m, PhH₅, 3-H), 6.38 (d,J = 16 Hz, 8-H), 6.14 (dd, J = 16.1 and 8.0 Hz, 7-H). 6.03 (d, J = 16 Hz, 2-H), 3.79 (q, J = 4.3 Hz, 5-H), 2.94 (brs, CH₂CH₂), 2.58-2.42 (m, 6-H, 4-HH'), 1.10 (d,J = 6.8 Hz, 6-Me), 0.90 (s, 9H, SiCMe₃), 0.05 (s, 6H, SiMe2) ppm; IR (CHCl₃) υₘₐₓ 2957, 2931, 2858, 1772, 1741, 1648, 1364, 1254, 1092, 1069, 838 cm⁻¹; MS (FD) 457 (M⁺,100); Anal. calcd. for C₂₅H₃₅NO₅ requires:
C,65.61;H,7.71;N,3.06%. Found: C,65.51;H,7.56; N, 3.02%.

### Preparation 4

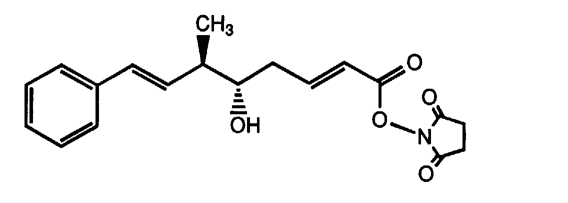

To a stirred solution of active ester of Preparation 3 (2.50g,5.47mmol) in CH₃CN (130mL) was added 48% aqueous HF (15mL) at 0C. The solution was stirred at 0 C for 0.75h and then at room temperature for 4h. The reaction was diluted with diethylether (300mL) and washed with H₂O until the wash was ∼pH7. Organics were dried (MgSO₄) and concentrated in vacuo to give a yellow residue which was recrystallized from Et2O to give alcohol as white crystals (1.46g,78%).
¹H NMR (CDCl₃) d 7.41-7.20 (m,PhH₅,3-H), 6.48 (d,J=16Hz,8-H), 6.15-6.07 (m,7-H,2-H), 3.71-3.65 (m,5-H), 2.83 (brs,CH₂CH₂), 2.60-2.33 (m,6-H,4-CH₂),1.95 (brs, 5-OH), 1.14 (d,J=6.8Hz,6-Me) ppm;
IR (KBr) υₘₐₓ 3457,1804,1773,1735,1724,1209,1099,1067, 1049,975,744,694 cm⁻¹;
UV (EtOH) λₘₐₓ 250 (ε =20535) nm;
MS (FD) 343.2 (M⁺,100);
[a]_{D} -57.8° (c 10.56, CHCI₃);
Anal. calcd. for C₁₉H₂₁NO₅S requires:
C,66.46;H,6.16;N,4.08%. Found: C,66.49; H,6.16; N, 4.07%.

### Preparation 5

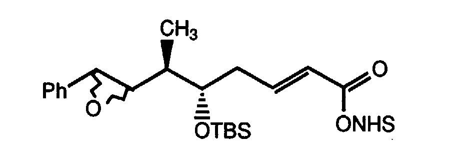

Acetone (10mL) was added to a solution of the active ester of Preparation 3 (2.90 g, 6.35 mmol) in dichloromethane (20 mL) and the solution cooled to 0°C. An aqueous solution of oxone (11.7 g, 19 mmol) in H₂O (30 mL) was slowly added to stirred solution of aqueous NaHCO₃ (5.3 g, 63.5 mmol) in H₂O (30 mL) (gas evolution observed!).The resulting solution was added to the reaction mixture and stirred at 0°C for 7h (tlc- 50% conversion). Further oxone (6 g) and acetone (15 mL) were added and the mixture stirred for 1.5h (tlc- all SM consumed). The reaction mixture was diluted with H₂O (5 volumes) and product extracted with CH₂Cl₂ (5 X 100mL). Combined, dried (MgSO₄) organics were concentrated in vacuo to give product as a yellow gummy solid (2.88 g). Tlc and ¹H NMR indicated 90% desired epoxide product (α:β =1:1.62): 10% SM. Crude product was purified by column chromatography (SiO₂: gradient elution: 15%-25% EtOAc: Hexanes) to give recovered styrene (389 mg, 13%) and epoxide as a yellow oil (2.38 g, 80%). Epoxides (2 g, α:β =1:1.50) were separated by HPLC to give β-epoxide as a white crystalline solid (1.17 g, 59 %. 99.8 % de) and a-epoxide as white crystalline solid (0.864 g, 43.2 %, 99% ee).

### Preparation 5A

### Alternative Preparation

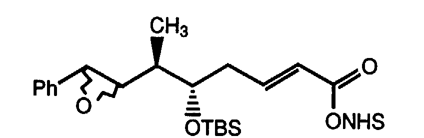

To a vigorously stirred solution of styrene (229mg, 0.50mmol) in acetonitrile (7.5 mL) was added 0.1M aqueous Na₂EDTA (5mL) and catalytic aqueous 0.1M tetra-n-butyl ammonium hydroxide (0.1 mL) at 0°C. A mixture of oxone (770mg, 1.25 mmol) and sodium bicarbonate (326 mg, 3.88 mmol) were pulverized and a portion (∼1/5) was added to the reaction mixture to bring the pH to ∼7. After 5 min, ketone (194 mg, 0.752 mmol) was added portion wise over a 1 h period. Simultaneously, the remaining Oxone - sodium bicarbonate mixture was added over ∼1h. After the additions were completed, the reaction was allowed to be stirred at 0°C for 4.5 h (HPLC showed styrene: epoxide 50:50 and α:β epoxide = 1: 5.6). Further Oxone (380 mg) and sodium bicarbonate (170 mg) were added portionwise over 1 h and then the reaction allowed to stir for a further 3.5 h. the reaction was diluted with ethyl acetate (50 mL) and washed with water (50 mL). Organics were dried (MgSO₄) and concentrated in vacuo to give crude product as an oil (140 mg).
HPLC: Reverse phase C18 column, 70:30 CH₃CN:H₂O, flow rate 1.0 mL/min, 1=220 nm
β-epoxide Rt=6.80 min (38.3%), α-epoxide Rt=8.43 min (8.71%), styrene Rt=13.90 min (2.81%).
α:β epoxide = 1:4.4 and styrene: epoxide = 6:94

### Preparation 6

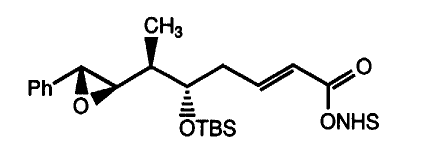

HPLC: C18 reverse phase, flow rate lmL/min, 60:40-CH₃CN:H₂O, λₘₐₓ = 254 nm, β-epoxide Rt=17.2 mins (AUC 1.5); [α]_{D}⁵⁸⁹ +77.36 (c 1.06, CH₂Cl₂); ¹H NMR (CDCl₃) δ 7.35-7.24 (m, 6H, ArH₅, 3-H), 6.08 (d, J= 15.8 Hz, 2-H), 3.91-3.88 (m, 5-H), 3.70 (s, 8-H), 2.97 (dd, J=6 and 0.9 Hz, 7-H), 2.85 (s, 4H, CH₂CH₂), 2.56-2.51 (m, 4-HH'), 1.78-1.76 (m, 6-H), 1.06 (d, J=6.9 Hz, 6-Me), 0.86 (s, 6H, SiCMe₃), 0.05 (s, SiMe), 0.01 (s, SiMe) ppm; IR (CHCl₃) υₘₐₓ 2957, 2931, 1742, 1773, 1200, 1069, 839 cm⁻¹; UV (EtOH) λₘₐₓ 217 (e = 21180) nm; MS (FD) *m/z* 474 (M⁺, 10), 416 ([M-CMe₃] ⁺, 100); Anal. calcd. for C₂₅H₃₅NO₆ requires: C, 63.40; H, 7.45; N, 2.96 %. Found: C, 63.45; H, 7.31; N, 3.21 %.

### Preparation 7

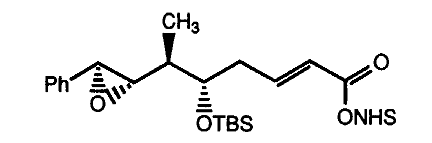

HPLC: C18 reverse phase, flow rate 1mL/min, 60:40-CH₃CN:H₂O, λₘₐₓ =254nm, α-epoxide Rt=21.0 mins (AUC 1.0); [α]_{D}⁵⁸⁹ +10.68° (c 1.03, CH₂Cl₂); ¹H NMR (CDCl₃) δ 7.38-7.26 (m, 6H, ArH₅, 3-H), 6.13 (d, J = 15.7 Hz, 2-H), 3.94-3.89 (m, 5-H), 3.60 (s, 8-H), 2.99 (dd, J= 7.3 and 1.3 Hz, 7-H), 2.85 (s, 4H, CH₂CH₂), 2.76-2.71 (m, 4-H), 2.61-2.54 (m, 4-H'), 1.64 (dt, J = 7.2 and 2.8 Hz, 6-H), 1.03 (d, J = 7 Hz, 6-Me), 0.90 (s, 9H, SiMe₃), 0.08 (s, SiMe), 0.05 (s, SiMe) ppm; IR (CHCl₃) υₘₐₓ 2957, 2931, 1741, 1773, 1649, 1254, 1200, 1125, 1095, 1069, 891, 839 cm⁻¹; UV (EtOH) λₘₐₓ 218 (ε = 21727) nm; MS (FD) *m/z* 474 (M⁺, 10), 416 ([M-CMe₃]⁺, 100); Anal. calcd. for C₂₅H₃₅NO₆ requires: C, 63.40; H, 7.45; N, 2.96%. Found: C, 63.20; H, 7.63; N, 3.07%.

### Preparation 8

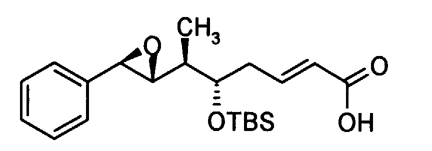

### Preparation of β-Epoxy Fragment A Acid (3c'). A solution of 2a' (1.91 g, 5.30 mmol) of the formula

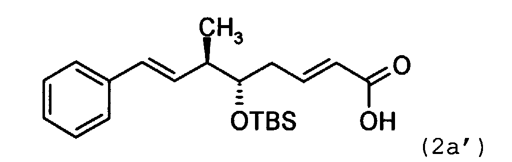

in CH₂Cl₂ (18 mL) was treated with *m*-chloroperbenzoic acid (0.96 g, 5.6 mmol) and the mixture stirred for 4 h before the volatiles were evaporated to give a colorless oil (2.88 g). Preparative HPLC was used to separate the epoxides (1.2:1 β:α) to give the desired β-epoxide as a colorless solid (42%). ¹H NMR (500 MHz, CDCl₃) δ 7.37-7.27 (m, 5H), 7.11 (ddd, 1H, J = 15.5, 7.6, 7.6 Hz), 5.92 (d, 1H, J = 15.5 Hz), 3.90 (ddd, 1H, J = 5.6, 5.6, 5.4 Hz), 3.70 (d, 1H, J = 2.0 Hz), 3.00 (dd, 1H, J = 6.6, 2.1 Hz), 2.51 (dd, 2H, J = 6.5, 6.5 Hz), 1.77-1.73 (m, 1H), 1.10 (d, 3H, J = 6.8 Hz), 0.89 (s, 9H), 0.07 (s, 3H), 0.03 (s, 3H). MS (FD) *m/z* 377 (M+1, 43), 319 (M-57, 100).

### Preparation 9

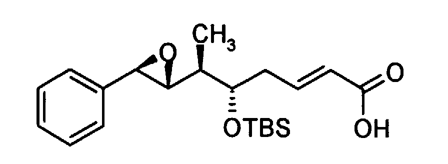

**Alternate Preparation of β-Epoxy Fragment A Acid.** To a stirred solution of acid **2a'** (100 mg, 0.277 mmol) in CH₃CN (3.7 mL) at 0°C was added a solution of Na₂EDTA (1X10⁻⁴ M in H₂O, 2.8 mL, 0.28 µmol) and tetrabutylammonium hydroxide (1 M in MeOH, 28 µL, 28 µmol). After NaHCO₃ (23.3 mg, 0.277 mmol) was added, the pH was adjusted to 8.0 with 2 M NaOH and a mixture of Oxone (1.70 g, 2.77 mmol) and NaHCO₃ (722 mg, 8.59 mmol) prepared. A 100 mg portion of the Oxone/NaHCO₃ was added followed by ketone **(2b)** (143 mg, 0.554 mmol). The pH was immediately adjusted to 7.8-8.0 with 2 M NaOH. The rest of the Oxone/ NaHCO₃ mixture was added in 95 mg portions in 10 min intervals and a solution of **(2b)** (143 mg, 0.554 mmol) in CH₃CN (500 µL) was added to the mixture during this period via a syringe pump. Throughout the experiment the pH was maintained at 7.8-8.0 with 2 M NaOH and 1 N H₂SO₄. HPLC analysis (C18 reverse phase, detection at 220 nm, flow rate at 1 mL/min, CH₃CN (0.05% TFA) / H₂O (0.05% TFA) - % CH₃CN: 80% to 90% over 10 min) 3 h after the Oxone addition revealed that the conversion was greater than 95% with a β/α epoxide ratio of 5.0:1. The mixture was filtered and the wetcake washed with CH₂Cl₂ (15 mL). The filtrate was washed with H₂O (15 mL) and the aqueous phase back extracted with CH₂Cl₂ (15 mL). The combined organic phases were washed with 0.1 M HCl (10 mL) and H₂O (10 mL), dried (MgSO₄), and concentrated to give the crude product^{4a} as a yellow oil (104 mg, 100%).

### Preparation 10

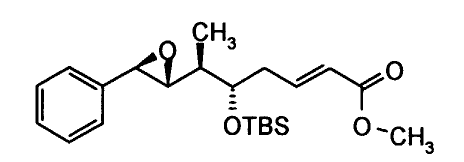

**Preparation of β-Epoxy Fragment A Methyl Ester.** The epoxidation of the methyl ester of Preparation 1, step 10 (104 mg, 0.278 mmol) was performed in the same manner as described in Preparation 9 except that the pH was lowered to 3.3 with 1 N H₂SO₄ after the tetrabutylammonium hydroxide was added, prior to the addition of sodium bicarbonate. HPLC analysis (same method as used for the analysis of the product of Preparation 9 except % CH₃CN: 95%, isocratic) 2 h after the Oxone addition revealed that conversion was greater than 95% with a β/α epoxide ratio of 4.9:1. After CH₂Cl₂ (6 mL) was added, the mixture was filtered and the wetcake washed with CH₂Cl₂ (14 mL). The filtrate was washed with H₂O (10 mL) and the aqueous phase back extracted with CH₂Cl₂ (2 X 20 mL). The combined organic phases were dried (MgSO₄) and concentrated to give the crude product as a yellow oil (123 mg, 113%). ¹H NMR (500 MHz, CDCl₃) δ 7.38-7.26 (m, 5H), 6.99 (ddd, 1H, J = 15.8, 7.6, 7.6 Hz), 5.91 (d, 1H, J = 15.8 Hz), 3.87 (ddd, 1H, J = 5.6, 5.6, 5.4 Hz), 3.75 (s, 3H), 3.70 (d, 1H, J = 2.1 Hz), 3.00 (dd, 1H, J = 6.8, 2.1 Hz), 2.49-2.45 (m, 2H), 1.75-1.69 (m, 1H), 1.10 (d, 3H, J = 6.8 Hz), 0.88 (s, 9H), 0.06 (s, 3H), 0.02 (s, 3H). MS (FD) *m/z* 391 (M+1, 8), 333 (M-57, 100).

### Preparation 11

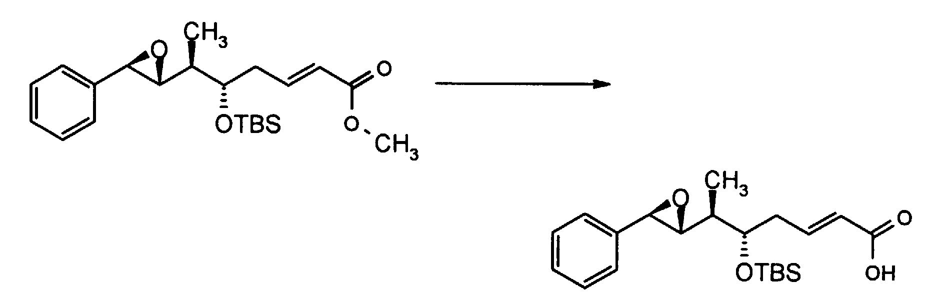

**Alternate Preparation of β-Epoxy Fragment A.** To a solution of the methyl ester of Preparation 10 (7.35 g, 18.8 mmol) in 35 mL of tetrahydrofuran was added 35 mL of 2N potassium hydroxide. The biphasic mixture was allowed to stir at 56°C for 14 h. Upon cooling to room temperature the layers were separated and the aqueous layer was washed with t-butyl methyl ether (1 x 50 mL). The combined organics were washed with 1N hydrochloric acid (1 x 35 mL) followed by brine (1 x 35 mL). Drying (Na₂SO₄) with simultaneous Darco (20-40 mesh) treatment followed by filtration and concentration *in vacuo* provided 7.85 g of the crude acid as a brown oil.

### Example 1

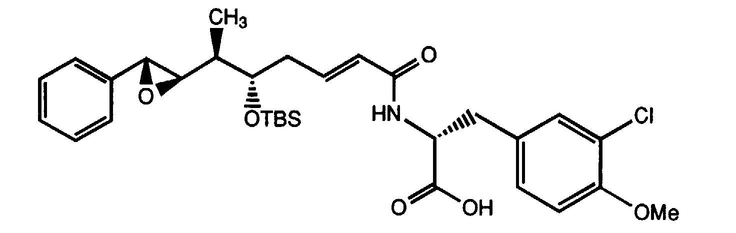

To a solution of β-epoxide of Preparation 6 (473mg, 1.0mmol) in dry DMF (6.7mL) was added amino acid "B" (459mg, 2.0mmols), represented by the formula 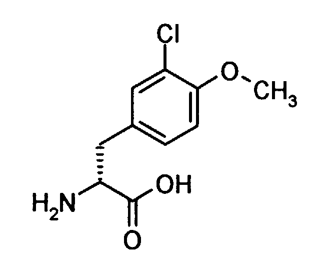 PCT Intnl. Publ. No. WO 97/07798, published March 6, 1997; followed by N,O-bis-(trimethylsilyl)acetamide (618uL, 2.5mmols) at room temperature under a nitrogen atmosphere. The resulting mixture was heated at 55°C (solution formed) for 8h, diluted with EtOAc (250mL) and washed with 1N aqueous HCl (3x80mL), H₂O (100mL). Combined, dried (MgSO₄) organics were concentrated in vacuo to give a yellow foam (590mg), which further purified by column chromatography (SiO₂, gradient elution; CH₂Cl₂ - 5%-10% MeOH: CH₂Cl₂) to give silyl ether product as white foam (489 mg, 89%). [α]_{D}⁵⁸⁹ +28.33° (c 1.06, MeOH); ¹H NMR (DMSO-*d*6)δ **Unit A:** 7.33-7.17 (m, ArH₅), 6.55-6.40 (m, 3-H), 6.03 (d, J = 15.3 Hz, 2-H), 3.83-3.76 (m, 5-H), 3.71 (s, 8-H), 2.90 (d, J = 6.8 Hz, 7-H), 2.46-2.27 (m, 4-HH'), 1.50-1.44 (m, 6-H), 0.94 (d, J = 6.7 Hz, 6-Me), 0.74 (s, 9H, SiMe₃), -0.54 (s, SiMe), -0.13 (s, SiMe); **Unit B:** 7.76 (d, J = 7.3, NH), 7.33-7.17 m, ArH), 7.04 (d, J = 8.5, ArH), 6.90 (d, J = 8.5, ArH), 4.27-4.23 (m, 2-H), 3.72 (s, 3H, OMe), 3.02 (dd, J = 13.3 and 4.3 Hz, 3-H), 2.78 (dd, J = 13.5 and 7.8 Hz, 3-H') ppm; IR (KBr) u 2955, 2930, 2857, 1668, 1605, 1504, 1463, 1454, 1279, 1258, 1067, 1026, 837, 776 cm⁻¹; UV (EtOH) lₘₐₓ 278 (e = 2219) nm.

### Example 2

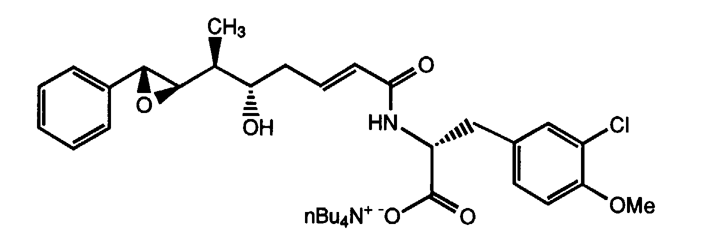

### Method A

To a solution of silyl ether of Example 1 (160mg, 0.272mmols) in dry DMF (3.5mL) was added sodium bicarbonate (228mg, 2.72mmols) followed by solid tetrabutylammonium fluoride-hydrate (TBAF) (358mg, 1.36mmols). The mixture was heated at 60°C for 17h and then further TBAF (358mg, 1.36mmols) and heated for 9h and finally a solution of 1M TBAF in THF (360uL, 1.36mmols) added turning the reaction a brown colour. The mixture was heated for 20 mins and then the reaction quenched in water (100mL) and extracted with EtOAc (3x50mL). Combined, dried (Na₂SO₄) organics were concentrated in vacuo to give a brown oily gum (248mg). Crude carboxylate salt was used in the next step without further purification.

### Example 3

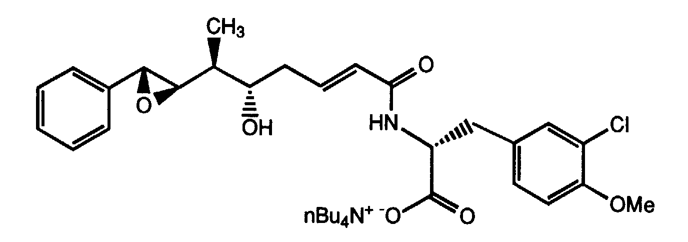

### Method B

To a solution of silyl ether of Example 1 (145mg, 0.247mmols) in dry tetrahydrofuran (3.0mL) was added a 1M solution of tetrabutylammonium fluoride (800uL, 0.8mmols) under a dry nitrogen atmosphere. The resulting solution was heated at 60°C for 7h and then worked-up as described above to give a brown residue (166mg, 94%). Crude carboxylate salt was used in the next step without further purification.

### Example 4

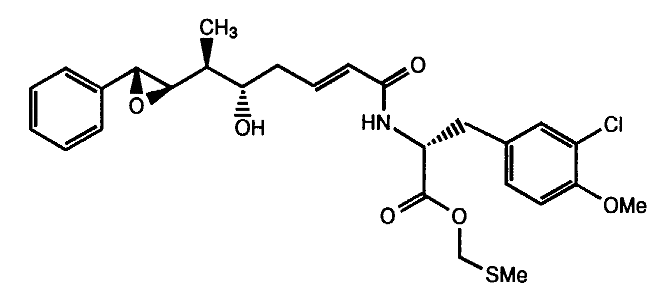

To a dry solution of crude carboxylate salt (0.272mmols) in DMSO (3.5mL) was sodium bicarbonate (274mg, 3.26mmols) followed by slow addition of a solution of t-butyl bromide (373mg, 2.72mmols) in DMSO (1.5mL) over ∼2h at room temperature and under nitrogen. The mixture was stirred for a further 21h and then quenched in brine (50mL) and extracted with EtOAc (3x 30mL). Combined organics were washed with water (50mL), dried (Na₂SO₄) and concentrated in vacuo to give crude ester as a gummy solid (117mg, 81%). The crude alcohol A-B was used in the next step without further purification.

### Example 5

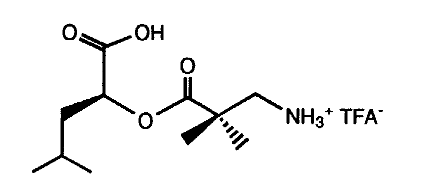

Boc amine (1.69g,5.09mmols) of the formula 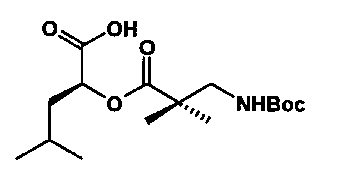 PCT Intnl. Publ. No. WO 97/07798, published March 6, 1997; was dissolved in trifluoroacetic acid (17ml) and the solution stirred at room temperature under a dry nitrogen atmosphere for 4.75h and then concentrated in vacuo and dried under high vacuum for 24h to give the amine salt as a yellow viscous oil (1.76g, 100%).
[α]_{D}⁵⁸⁹ -11.54° (c 1.04, MeOH); ¹H NMR (CDCl₃) δ **Unit C'** : 7.43 (br s, 3H, NH₃⁺),3.34-3.28 (m, 3-H), 3.18-3.12 (m, 3-H'), 1.42 (s, 2-Me), 1.36 (s, 2-Me); **Unit D:** 10.94 (br s, CO₂H), 5.23-5.20 (m, 2-H),1.92-1.77 (m, 3H, 3-HH', 4-H), 1.10 (d, J = 5.8 Hz, 5-H3), 0.98 (d, J = 5.8 Hz, 4-Me) ppm; IR (CHCl₃) υ 2963, 1746, 1710, 1678, 1192, 1172 cm⁻¹; MS (FAB) 232.2 ([M+1]⁺, 100).

### Example 6

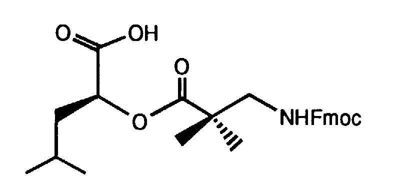

To a stirred solution of amine salt of Example 5 (5.09mmols) in dioxane (20mL) was added sodium bicarbonate (2.14g,25.5mmols) followed by FmocCl (1.58g,6.11mmols) at room temperature. The mixture was diluted with H₂O (4mL) and stirred for 19h. The reaction mixture was quenched in 1N aqueous HCl (150mL) and extracted with EtOAc (2x100mL). Combined organics were washed with H₂O (100mL), dried (MgSO₄) and concentrated in vacuo to give a yellow gummy solid. The crude product was purified by column chromatography (Biotage-SiO₂: gradient elution; 10%- 75% EtOAC: Hexanes) to provide Fmoc amine as a pale yellow solid (850mg, 37%). Product was contaminated with amino acid, which was removed by dissolving the product in EtOAc and stirring with 1N HCl aq for several hours. Organics were dried and concentrated to give product (85:15 product: amino acid).
[α]_{D}⁵⁸⁹ -15.95° (c 0.50, CH₂Cl₂); ¹H NMR (CDCl₃) δ **Unit C'** : 7.59 (d, J = 7.4 Hz, ArH₂), 7.67-7.61 (m, ArH₂), 7.43 (t, J = 7.3 Hz, ArH₂), 7.36- 7.30 (m, ArH₂), 5.88 (t, J = 5.8 Hz, NH), 4.41-4.38 (m, 3'-HH'), 4.35-4.28 (m,4'-H), 3.42 (d, J = 6.5 Hz, 3-HH'), 1.27 (s, 2Me), 1.26 (s, 2-Me); **Unit D**: 8.40 (br s, CO2H), 5.18-5.13 (m, 2-H), 1.87-1.69 (m, 3H, 3-HH', 4-H), 0.97 (d, J = 5,8 Hz, 5-H3), 0.93 (d, J = 6.1 Hz, 4-Me) ppm; IR (KBr) ν 2959, 2937, 1730, 1540, 1471, 1451, 1307, 1268, 1145, 1128, 759, 741 cm⁻¹; UV (EtOH) λₘₐₓ 299 (e = 5851), 288 (e = 4773), 265 (e = 18369), 227 (e = 4813) nm; MS (FAB) 454 ([M+1]⁺, 26); Anal. calcd. for C₂₆H₃₁NO₆ requires: C, 68.86; H, 6.89; N, 3.09%. Found: C, 68.92; H, 7.01; N, 3.34%.

### Example 7

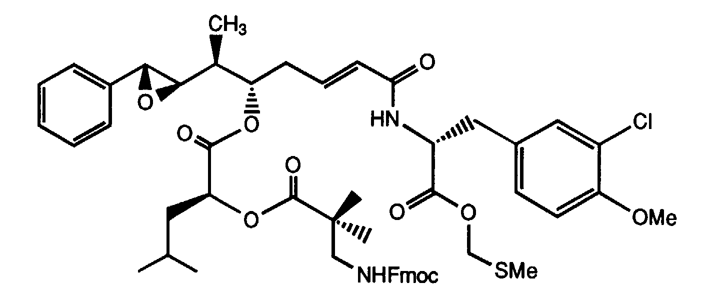

To a stirred solution of carboxylic acid D-C' of Example 6 (129mg, 0.285mmols) in dry dichloromethane (1.0mL) was added DMAP (5.4mg, 0.044mmols) and DCC (59mg, 0.285mmols) at room temperature under a dry nitrogen atmosphere. The solution was stirred for 0.5h and then solid sodium bicarbonate (37mg, 0.44mmols) added followed by a solution of crude alcohol A-B of Example 4 (117mg, 0.22mmols) in dry dichloromethane (1.2mL). A precipitate formed within 10 mins and the mixture was stirred for a further 50 mins. The crude reaction mixture was directly applied onto a SiO₂ colum and purified (gardient elution; 10%-40% EtOAc:Hexanes) to give methyl sulphide product as pale yellow solid (122mg, 46% over 3 steps).
¹H NMR (CDCl₃) δ **Unit A**: 7.43-7.20 (m, ArH₅), 6.90-6.81 (m, 2H, 3-H, ArH), 5.93 (d, J = 15.6 Hz, 2-H), 5.14-4.93 (m, 5-H), 3.05 (dd, J = 14.5 and 8.3 Hz, 7-H), 2.65- 2.63 (m, 4-HH'), 2.00-1.95 (m, 6-H), 1.17 (d, J = 7.0, 6-Me); **Unit B**: 7.43-7.20 (m, ArH), 7.06 (d, J = 8.1 Hz, ArH), 6.90-6.81 (m, ArH), 6.44 (d, J = 7.7 Hz, NH), 5.19 (q, J_{AB} = 11.8 Hz, 1'-HH), 5.14-4.93 (m, 2-H), 3.87 (s, OMe), 3.20-3.10 (m, 3-HH'), 2.21 (s, SMe); **Unit C'**: 7.79 (d, J = 7.4 Hz, ArH₂), 7.67 (d, J = 6.9 Hz, ArH₂), 7.43-7.20 (m, ArH₄), 6.04 (d, J = 7.7 Hz, NH), 4.42-4.34 (m, 3'-HH'), 4.30-4.25 (m, 4'-H, 3.42 (d, J = 6.2 Hz, 3-HH'), 1.27 (s, 2-Me), 1.20 (s, 2-Me); **Unit D**: 5.22-5.18 (m, 2-H), 1.82-1.58 (m, 3H, 3-HH',4-H), 0.96 (s, 5-H3), 0.94 (s, 4-Me) ppm.

### Example 8

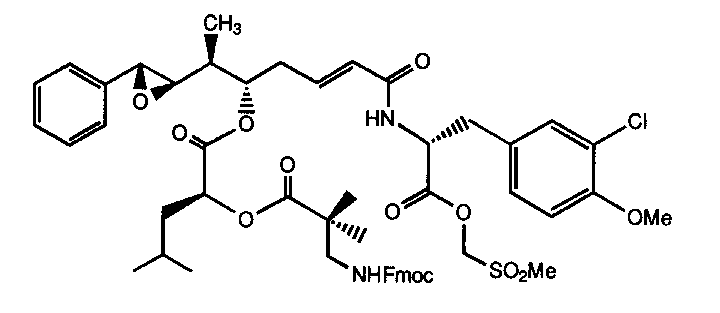

To a stirred solution of methyl sulphide of Example 7 (56mg,0.058mmols) in acetone (10mL) was added sodium bicarbonate (64mg, 0.764mmols) followed by an aqueous solution of oxone (234mg, 0.382mmols) in water (3.0mL). The reaction mixture was stirred at room temperature for 20 mins (SM is rapidly converted to a very polar component sulphoxide and then with time to the less polar sulphone product). The reaction was quenched in water (40mL) and extracted with EtOAc (3x20mL). Organics were washed with brine (30mL), dried (MgSO₄) and concentrated in vacuo to give a solid. Crude product was purified by column chromatography (SiO₂: gradient elution; 25%-60% EtOAc:Hexanes) to give sulphone as a white foamy solid (43mg, 74%).
¹H NMR (CDCl₃) δ **Unit A**: 7.58-7.17 (m, ArH₅), 6.82-6.75 (m, 3-H), 5.87 (d, J = 16Hz, 2-H), 4.98-4.86 (m, 5-H), 3.70 (d, J = 1.1 Hz, 7-H), 2.92-2.89 (m,7-H), 2.61-2.58 (m,4-HH'), 1.94-1.89 (m, 6-H), 1.13 (d, J = 7.1 Hz, 6-Me); **Unit B**: 7.58-7.17 (m, ArH), 7.04 (d, J = 7.7 Hz, ArH), 6.81 (d, J = 8.1 Hz, ArH), 6.54 (d, J = 7.5 Hz, NH), 4.98-4.86 (m, 2-H), 3.84 (s, 7-OMe), 3.17-2.98 (dq, J_{AB} = 14 and 6.6 Hz, 2-HH'); **Unit C'**: 7.75 (d, J = 7.4 Hz, ArH₂), 7.62 (d, J = 6.8 Hz, ArH₂), 7.58-7.17 (m, ArH₄), 5.97 (t, J = 5.5 Hz, NH), 5.00 (s, SO₂Me), 4.98-4.86 (m, 2H, 1'-HH'), 4.38-4.33 (m, 3'-HH'), 4.25-4.20 (m, 4'-H), 3.40-3.36 (m, 3-HH'), 1.22 (s, 2-Me), 1.15 (s, 2-Me); **Unit D**: 5.19 (q, J_{AB} = 5 Hz, 2-H), 1.80-1.61 (m, 2H, 3-H, 4-H), 1.57-1.49 (m, 3-H'), 0.91 (s, 5-H₃), 0.89 (s, 4-Me) ppm.

### Example 9

### Cryptophycin 52

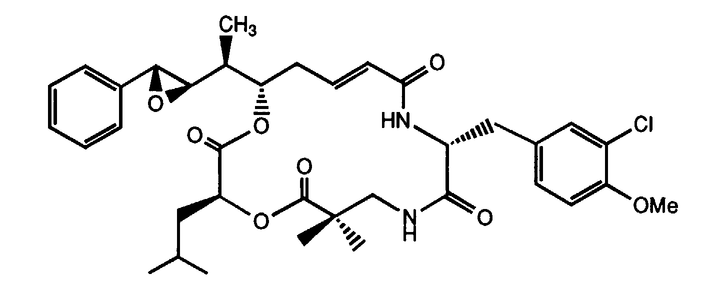

To a stirred solution of sulphone of Example 8 (18mg, 17.98umols) in dry DMF (2.0mL) was added neat piperidine (8.9uL, 90umols) at room temperature and under nitrogen. The resulting solution was stirred for 5h and then concentrated in vacuo to give crude amine as a foam. The amine was dissolved in toluene (3 mL) and heated at 60°C under nitrogen for 40mins. The reaction solution was directly purified by column chromatography (SiO₂; gradient elution; 20%-75% EtOAc:Hexanes) to give cryptophycin 52 as a white glass (6.1mg, 51% over 2 steps).
¹H NMR (CDCl₃) δ **Unit A**: 7.45-7.38 (m, ArH₃), 7.31-7.23 (m, ArH₂), 6.85-6.76 (m, 3-H), 5.76 (d, J = 15.6 Hz, 2-H), 5.27-5.23 (m, 5-H), 2.97 (dd, J = 7.5 and 1.7 Hz, 7-H), 2.66-2.44 (m, 4-HH'), 1.86-1.67 (m, 6-H), 1.19 (d, J = 6.9 Hz, 6-Me); **Unit B**: 7.31-7.23 (m, ArH), 7.09 (dd, J = 8.3 and 2.0 Hz, ArH), 6.88 (d, J = 8.4 Hz, ArH), 5.50 (d J = 7.8 Hz, NH), 4.79 (q, J = 6.4 Hz, 2-H), 3.92 (s, OMe), 3.73 (d, J = 1.5 Hz, 8-H), 3.17-3.11 (m, 3-HH'); **Unit C'**: 3.47 (dd, J = 13.4 and 8.7 Hz, 3-H), 3.17-3.11 (m, 3'-H), 1.27 (s, 2-Me), 1.20 (s, 2-Me); **Unit D**: 4.87 (dd, J = 10 and 3.3 Hz, 2-H), 1.86-1.67 (m, 2H, 3-H, 4-H), 1.40-1.30 (m, 3-H'), 0.88 (app t, J = 6.3 Hz, 6H, 5-H3, 4-Me) ppm.

### Example 10

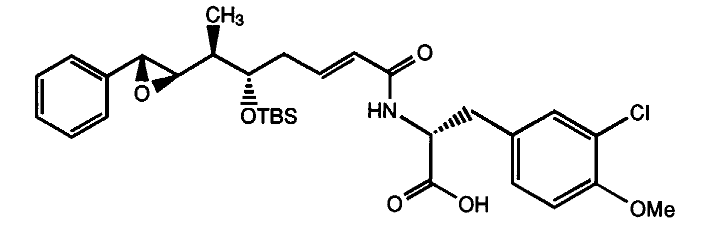

**Preparation of β-Epoxy Fragment A-B.** To a solution of the product of Preparation 8 (60.4 mg, 0.160 mmol) and N,N-diisopropylethylamine (56 µL, 0.32 mmol) in DMF (330 µL) was added the diphenylphosphinic chloride (32 µL, 0.17 mmol). After the mixture was stirred for 1 h, it was added to a mixture of amino acid "B", represented by the formula 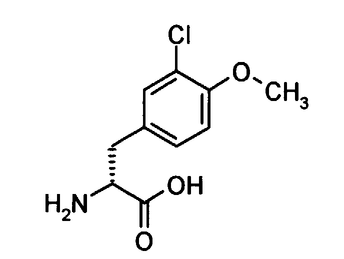 (40.4 mg, 0.176 mmol) and N,O-bis-(trimethylsilyl)-acetamide (99 µL, 0.40 mmol) in DMF (330 µL) that had been prepared 20 min earlier. The reaction mixture was stirred for 2 h before it was diluted with EtOAc (14 mL) and washed with 1 M HCl (3 X 4.5 mL) and H₂O (4.5 mL). The organic phase was dried (MgSO₄) and concentrated to a yellow oil. Chromatography on silica gel with 5 to 10% MeOH/CH₂Cl₂ gave the desired product as a colorless solid (83.2 mg, 88%).

### Example 11

### Alternative Preparation of Cryptophycin 52

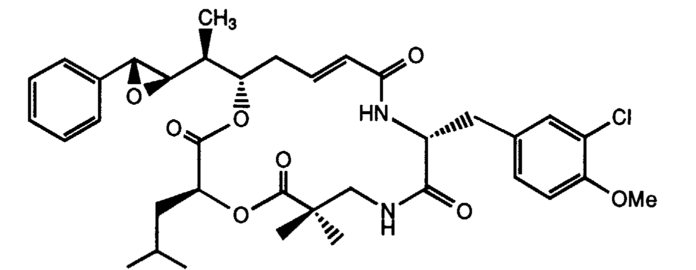

After a mixture of a compound of Example 7 (325 mg, 0.335 mmol) and piperidine (166 µL, 1.68 mmol) in DMF (34 mL) was stirred for 1 h at rt and 2 h at 60 °C, HPLC (C18 reverse phase, detection at 220 nm, flow rate at 1 mL/min, CH₃CN (0.05% TFA) / H₂O (0.05% TFA) - % CH₃CN: 60% to 95% over 25 min) revealed that the Fmoc protection had been removed and a mixture of the intermediate free amine and Cryptophycin 52 was now present. 2-Hydroxypyridine (63.7 mg, 0.670 mmol) was added and the reaction allowed to stir for 18 h before it was checked again by HPLC which showed that intermediate still remained. Additional piperidine (66 µL, 0.67 mmol) was added and the reaction stirred for another 64 h at which time HPLC showed that it was done. The mixture was diluted with EtOAc (90 mL) and washed with H₂O (3 X 90 mL). The combined aqueous phases were back extracted with EtOAc (30 mL) and the combined organic phases were dried (MgSO₄) and concentrated to an orange oil. Chromatography on silica gel with EtOAc/hexane (1:1 to 4:1) gave Cryptophycin 52 as a colorless solid (143 mg, 64% corrected to 58% due to contamination by N-formyl piperidine).

## Claims

1. A process for preparing a compound of the formula 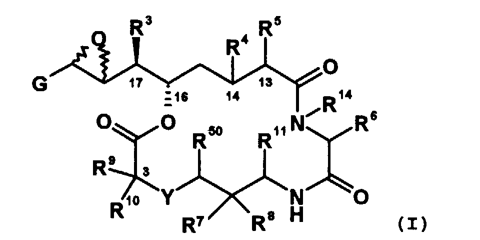 wherein
G is C₁-C₁₂ alkyl, C₂-C₁₂ alkenyl, C₂-C₁₂ alkynyl, or Ar;
Ar is an aromatic or heteroaromatic group or a substituted aromatic or heteroaromatic group;
R³ is C₁-C₆ alkyl;
R⁴ and R⁵ are each hydrogen; or R⁴ and R⁵ taken together form a second bond between C-13 and C-14;
R⁷ and R⁸ are each independently hydrogen or C₁-C₆ alkyl; or
R⁷ and R⁸ taken together form a cyclopropyl or cyclobutyl ring;
R⁹ is hydrogen, C₁-C₆ alkyl, C₂-C₆ alkenyl, C₂-C₆ alkynyl, - (CH₂)ₘ-(C₃-C₅) cycloalkyl or benzyl, wherein m is the integer one to three;
R¹⁰ is hydrogen or C₁-C₆ alkyl;
R¹¹ is hydrogen, C₁-C₆ alkyl, phenyl or benzyl;
R¹⁴ is hydrogen or C₁-C₆ alkyl;
R⁵⁰ is hydrogen or (=O);
Y is CH, O, NR¹², S, SO, SO₂, wherein R¹² is H or C₁-C₃ alkyl;
R⁶ is C₁-C₆ alkyl, substituted (C₁-C₆)alkyl, (C₃-C₈)cycloalkyl, substituted (C₃-C₈)cycloalkyl, a heteroaromatic or substituted heteroaromatic group or a group of formula (IA), (IB) or (IC): 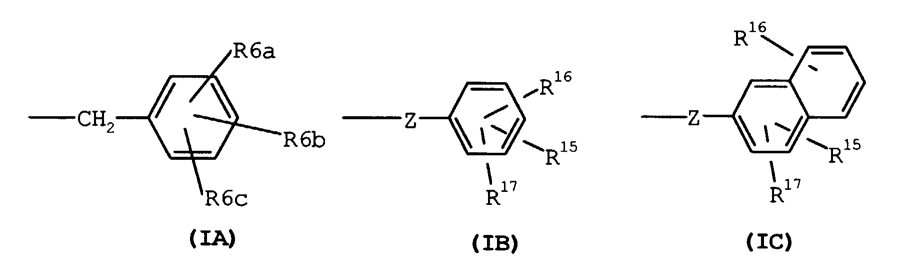
**R**^{**6a**}**, R**^{**6b**}**,** and **R**^{**6c**} independently are H, (C₁-C₆)alkyl, halo NR¹⁸R¹⁹ or OR¹⁸;
**R**^{**15**}**, R**^{**16**}**,** and **R**^{**17**} independently are hydrogen, halo, (C₁-C₆)alkyl, OR¹⁸, O-aryl, NH₂, NR¹⁸R¹⁹, NO₂, OPO₄H₂, (C₁-C₆ alkoxy)phenyl, S-benzyl, CONH₂, C0₂H, PO₃H₂, SO₂R²³, or Z';
**R**^{**18**} and **R**^{**19**} independently are hydrogen or C₁-C₆ alkyl;
**R**^{**23**} is hydrogen or (C₁-C₃)alkyl;
**Z** is -(CH₂)ₙ- or (C₃-C₅)cycloalkyl;
**n** is 0, 1, or 2; and
**Z'** is an aromatic or substituted aromatic group; or a pharmaceutically acceptable salt thereof;
comprising deprotecting a compound of the formula 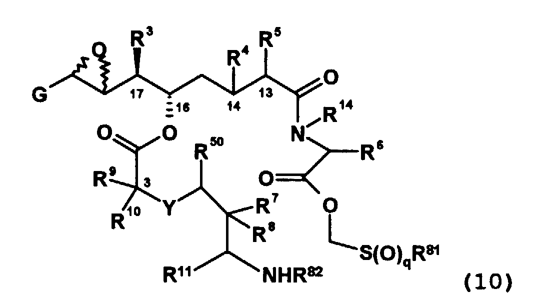 wherein G, R³, R⁵, R⁶, R⁷, R⁸, R⁹, R¹⁰, R¹¹, R¹⁴, R⁵⁰ and Y are as defined above; q is an integer 1 or 2; R⁸¹ is C₁-C₆ alkyl, C₃-C₈ cycloalkyl, phenyl or benzyl; and R⁸² is a base labile protecting group, with a deprotecting agent to form a compound of the formula 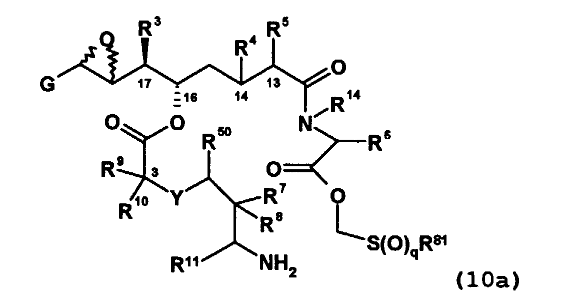 wherein G, R³, R⁵, R⁶, R⁷, R⁸, R⁹, R¹⁰, R¹¹, R¹⁴, R⁵⁰, Y, q and R⁸¹ are as defined above; optionally contacting the compound of formula (10a) with a cyclizing agent to form a compound of formula (I); and optionally forming a pharmaceutically acceptable salt thereof.

2. A process according to claim 1 wherein G is phenyl, para-fluorophenyl, or phenyl substituted with -CH₂OC(O) (CH₂)ₘ,NH₂; R³ is methyl; R⁴ and R⁵ taken together form a second bond between C-13 and C-14; R⁹ is C₁-C₆ alkyl; R¹⁰ is hydrogen; R¹¹ is hydrogen; R¹⁴ is hydrogen; R⁵⁰ is (=O); Y is O; and R⁶ is a group of the formula (IA).

3. A process according to claim 1 wherein said deprotecting agent is piperidine.

4. A process according to claim 2 wherein G is phenyl.

5. A process according to claim 1 wherein said compound of formula (I) is Cryptophycin 52.

6. A process for preparing a compound of the formula 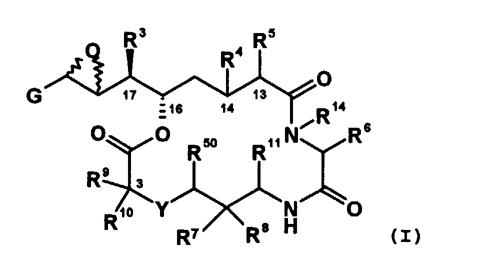 wherein
G is C₁-C₁₂ alkyl, C₂-C₁₂ alkenyl, C₂-C₁₂ alkynyl, or Ar;
Ar is an aromatic or heteroaromatic group or a substituted aromatic or heteroaromatic group;
R³ is C₁-C₆ alkyl;
R⁴ and R⁵ are each hydrogen; or R⁴ and R⁵ taken together form a second bond between C-13 and C-14;
R⁷ and R⁸ are each independently hydrogen or C₁-C₆ alkyl; or
R⁷ and R⁸ taken together form a cyclopropyl or cyclobutyl ring;
R⁹ is hydrogen, C₁-C₆ alkyl, C₂-C₆ alkenyl, C₂-C₆ alkynyl, -(CH₂)ₘ-(C₃-C₅)cycloalkyl or benzyl, wherein m is the integer one to three;
R¹⁰ is hydrogen or C₁-C₆ alkyl;
R¹¹ is hydrogen, C₁-C₆ alkyl, phenyl or benzyl;
R¹⁴ is hydrogen or C₁-C₆ alkyl;
R⁵⁰ is hydrogen or (=O);
Y is CH, O, NR¹², S, SO, SO₂, wherein R¹² is H or C₁-C₃ alkyl;
R⁶ is C₁-C₆ alkyl, substituted (C₁-C₆)alkyl, (C₃
C₈)cycloalkyl, substituted (C₃-C₈)cycloalkyl, a heteroaromatic or substituted heteroaromatic group or a group of formula (IA), (IB) or (IC): 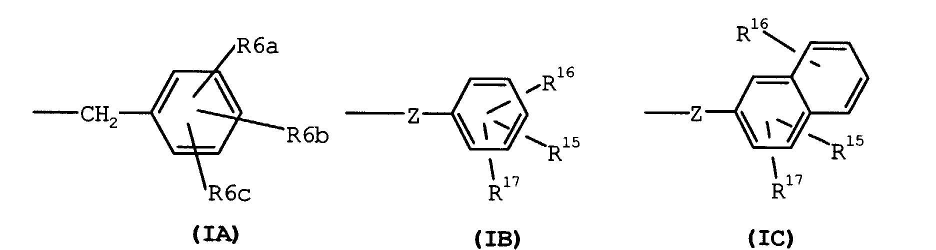
**R**^{**6a**}**, R**^{**6b**}**,** and **R**^{**6c**} independently are H, (C₁-C₆)alkyl, halo NR¹⁸R¹⁹ or OR¹⁸;
**R**^{**15**}**, R**^{**16**}**,** and **R**^{**17**} independently are hydrogen, halo, (C₁-C₆)alkyl, OR¹⁸, O-aryl, NH₂, NR¹⁸R¹⁹, NO₂, OPO₄H₂, (C₁-C₆ alkoxy)phenyl, S-benzyl, CONH₂, C0₂H, PO₃H₂, SO₂R²³, or Z';
**R**^{**18**} and **R**^{**19**} independently are hydrogen or C₁-C₆ alkyl;
**R**^{**23**} is hydrogen or (C₁-C₃)alkyl;
**Z** is -(CH₂)ₙ- or (C₃-C₅)cycloalkyl;
**n** is 0, 1, or 2; and
**Z'** is an aromatic or substituted aromatic group; or a pharmaceutically acceptable salt thereof; comprising the steps of
(a) epoxidizing a compound of the formula 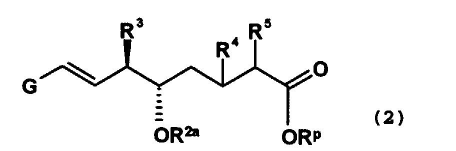 wherein G, R³, R⁴ and R⁵ are as defined above; R^{2a} is hydrogen or tri(C₁-C₆ alkyl)silyl; and R^{P} is hydrogen or a suitable activatable carboxy protecting group with an epoxidizing agent to form a compound of the formula 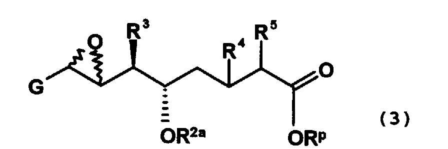 wherein G, R³, R⁴, R⁵,R^{2a} and R^{p} are as defined above, provided that R^{2a} and R^{p} are not both hydrogen;
(b) coupling the compound of formula (3) with an amino acid of the formula 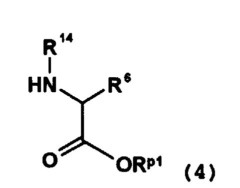 wherein R⁶ and R¹⁴ are as defined above and R^{p1} is hydrogen or C₁-C₆ alkyl; further in the presence of a silylating agent when R¹⁴ and R^{p1} are hydrogen; to yield a compound of the formula 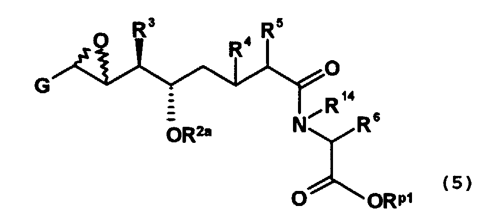 wherein G, R³, R⁴, R⁵, R^{2a}, R^{p1} R⁶ and R¹⁴ are as defined above;
(c) deprotecting the compound of formula (5) with a suitable alkoxy deprotecting agent and further carboxy-deprotecting the compound of formula (5) when R^{p1} is C₁-C₆ is alkyl, with a suitable base to form a compound of the formula 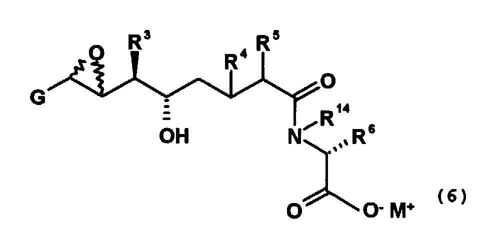 wherein G, R³, R⁴, R⁵, R⁶ and R¹⁴ are as defined above and M⁺ is a cation;
(d) contacting a compound of formula (6) with a thioester forming agent to form a compound of the formula 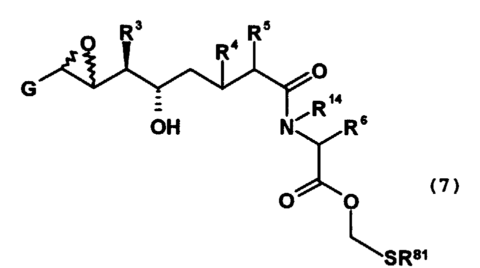 wherein G, R³, R⁴, R⁵, R⁶ and R¹⁴ are as defined above and R⁸¹ is C₁-C₆ alkyl, C₃-C₈ cycloalkyl, phenyl or benzyl;
(e) coupling a compound of formula (7) with a compound of the formula 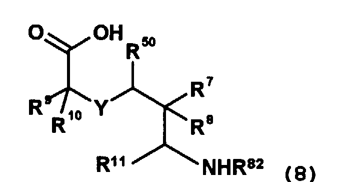 wherein R⁷, R⁸, R⁹, R¹⁰, R¹¹ and R⁵⁰ are as defined above and R⁸² is a base labile protecting group, to form a compound of the formula 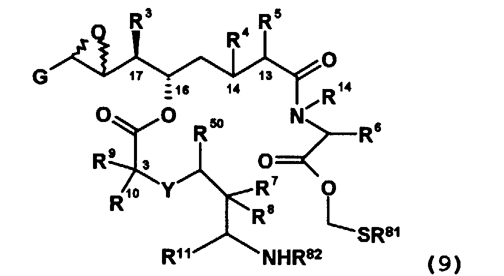 wherein G, R³, R⁴, R⁵, R⁶, R⁷, R⁸, R⁹, R¹⁰, R¹¹, R¹⁴, R⁵⁰ R⁸¹, R⁸² and Y are as defined above;
(f) oxidizing a compound of formula (9) with an oxidizing agent to form a compound of the formula 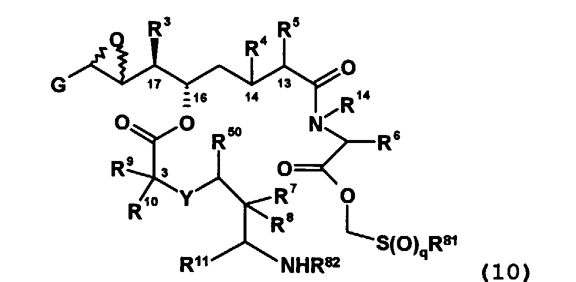 wherein G, R³, R⁴, R⁵, R⁶, R⁷, R⁸, R⁹, R¹⁰, R¹¹, R¹⁴, R⁵⁰, R⁸¹ and R⁸² and Y are as defined above and q is an integer 1 or 2;
(g) deprotecting a compound of formula (10) with a suitable deprotecting agent to form a compound of the formula 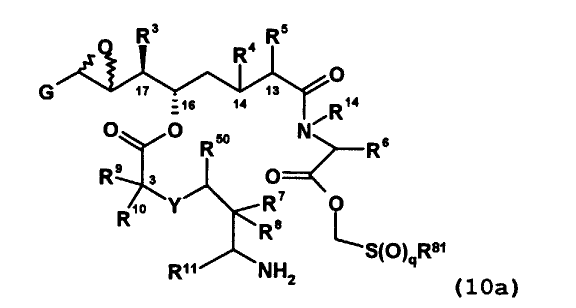 wherein G, R³, R⁵, R⁶, R⁷, R⁸, R⁹, R¹⁰, R¹¹, R¹⁴, R⁵⁰, Y, q and R⁸¹ are as defined above; and optionally contacting a compound of formula (10a) with a cyclizing agent to form a compound of formula (I); and
(h) optionally forming a pharmaceutically acceptable salt of a compound of formula (I).

7. A process according to claim 6 wherein G is phenyl, para-fluorophenyl, or phenyl substituted with -CH₂OC(O) (CH₂)ₘ,NH₂; R³ is methyl; R⁴ and R⁵ taken together form a second bond between C-13 and C-14; R⁹ is C₁-C₆ alkyl; R¹⁰ is hydrogen; R¹¹ is hydrogen; R¹⁴ is hydrogen; R⁵⁰ is (=O); Y is O; and R⁶ is a group of the formula (IA).

8. A process according to claim 6 wherein said deprotecting agent is piperidine.

9. A process according to claim 6 wherein said oxidizing agent is Oxone.

10. A process according to claim 6 wherein R⁸¹ is methyl.

11. A process according to claim 6 wherein said epoxidizing agent is Oxone in the presence of acetone or m-CPBA.

12. A process according to claim 6 wherein said epoxidizing agent is Oxone in the presence of a chiral ketone.

13. A process according to claim 12 wherein said chiral ketone is a compound of the formula

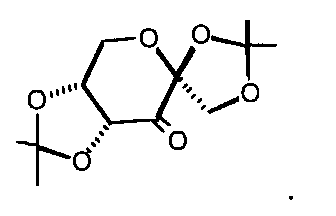

14. A process according to claim 7 wherein said deprotecting agent is piperidine; said oxidizing agent is Oxone; R⁸¹ is methyl; and wherein said epoxidizing agent is Oxone in the presence of acetone or *m*-CPBA.

15. A process according to claim 7 wherein said cyclizing agent is piperidine; said oxidizing agent is Oxone; R⁸¹ is methyl; and wherein said epoxidizing agent is Oxone in the presence of a chiral ketone of the formula

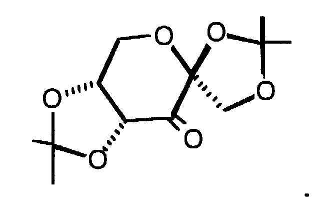

16. A process according to claim 6 wherein said compound of formula (I) is Cryptophycin 52.

17. A process according to claim 6 wherein R^{p} is hydrogen and said epoxidizing agent is m-chlorobenzoic acid.

18. A process according to claim 14 wherein R^{p} is hydrogen and said epoxidizing agent is m-chlorobenzoic acid.

19. A process according to claim 18 wherein said compound of formula (I) is Cryptophycin 52.

20. A process for preparing a compound of the formula 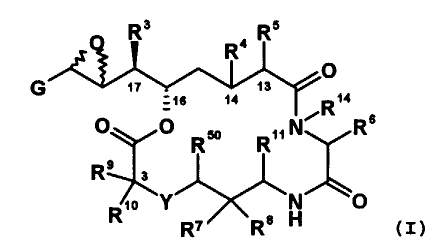 wherein
G is C₁-C₁₂ alkyl, C₂-C₁₂ alkenyl, C₂-C₁₂ alkynyl, or Ar;
Ar is an aromatic or heteroaromatic group or a substituted aromatic or heteroaromatic group;
R³ is C₁-C₆ alkyl;
R⁴ and R⁵ are each hydrogen; or R⁴ and R⁵ taken together form a second bond between C-13 and C-14;
R⁷ and R⁸ are each independently hydrogen or C₁-C₆ alkyl; or
R⁷ and R⁸ taken together form a cyclopropyl or cyclobutyl ring;
R⁹ is hydrogen, C₁-C₆ alkyl, C₂-C₆ alkenyl, C₂-C₆ alkynyl, -(CH₂)ₘ-(C₃-C₅)cycloalkyl or benzyl, wherein m is the integer one to three;
R¹⁰ is hydrogen or C₁-C₆ alkyl;
R¹¹ is hydrogen, C₁-C₆ alkyl, phenyl or benzyl;
R¹⁴ is hydrogen or C₁-C₆ alkyl;
R⁵⁰ is hydrogen or (=O);
Y is CH, O, NR¹², S, SO, SO₂, wherein R¹² is H or C₁-C₃ alkyl;
R⁶ is C₁-C₆ alkyl, substituted (C₁-C₆)alkyl, (C₃-C₈)cycloalkyl, substituted (C₃-C₈)cycloalkyl, a heteroaromatic or substituted heteroaromatic group or a group of formula (IA), (IB) or (IC): 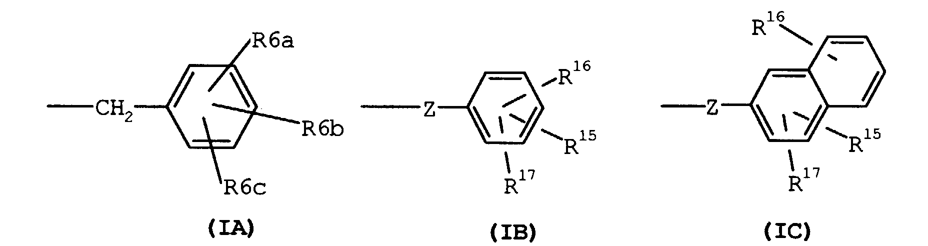
**R**^{**6a**}**, R**^{**6b**}**,** and **R**^{**6c**} independently are H, (C₁-C₆)alkyl, halo NR¹⁸R¹⁹ or OR¹⁸;
**R**^{**15**}**, R**^{**16**}**,** and **R**^{**17**} independently are hydrogen, halo, (C₁-C₆)alkyl, OR¹⁸, O-aryl, NH₂, NR¹⁸R¹⁹, NO₂, OPO₄H₂, (C₁-C₆ alkoxy)phenyl, S-benzyl, CONH₂, C0₂H, PO₃H₂, SO₂R²³, or Z';
**R**^{**18**} and **R**^{**19**} independently are hydrogen or C₁-C₆ alkyl;
**R**^{**23**} is hydrogen or (C₁-C₃)alkyl;
**Z** is -(CH₂)ₙ- or (C₃-C₅)cycloalkyl;
**n** is 0, 1, or 2; and
**Z'** is an aromatic or substituted aromatic group; or a pharmaceutically acceptable salt thereof; comprising deprotecting a compound of the formula 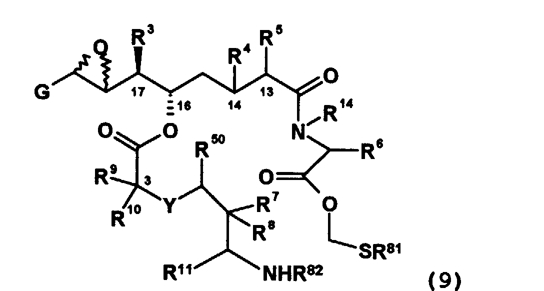 wherein G, R³, R⁴, R⁵, R⁶, R⁷, R⁸, R⁹, R¹⁰, R¹¹, R¹⁴, R⁵⁰, and Y are as defined above, R⁸¹ is C₁-C₆ alkyl, C₃-C₈ cyclolkyl, phenyl or benzyl, and R⁸² is a base labile protecting group; with a deprotecting agent to form a compound of the formula 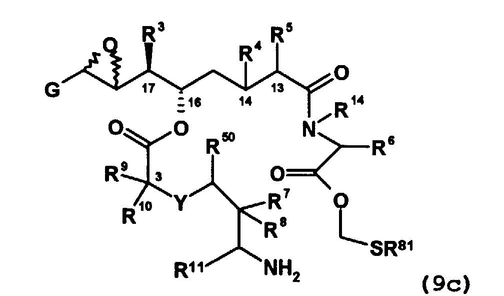 wherein G, R³, R⁴, R⁵, R⁶, R⁷, R⁸, R⁹, R¹⁰, R¹¹, R¹⁴, R⁵⁰ and Y are as defined above; optionally contacting a compound of formula (9c) with a cyclizing agent to form a compound of formula (I); and optionally forming a pharmaceutically acceptable salt thereof.
to form a compound of formula (I) and optionally forming a pharmaceutically acceptable salt thereof.

21. A process according to claim 20 wherein G is phenyl, para-fluorophenyl, or phenyl substituted with -CH₂OC(O) (CH₂)ₘ,NH₂; R³ is methyl; R⁴ and R⁵ taken together form a second bond between C-13 and C-14; R⁹ is C₁-C₆ alkyl; R¹⁰ is hydrogen; R¹¹ is hydrogen; R¹⁴ is hydrogen; R⁵⁰ is (=O); Y is O; and R⁶ is a group of the formula (IA).

22. A process according to claim 20 wherein said deprotecting agent is piperidine.

23. A process according to claim 20 wherein G is phenyl.

24. A process according to claim 20 wherein said compound of formula (I) is Cryptophycin 52.

25. A compound of the formula 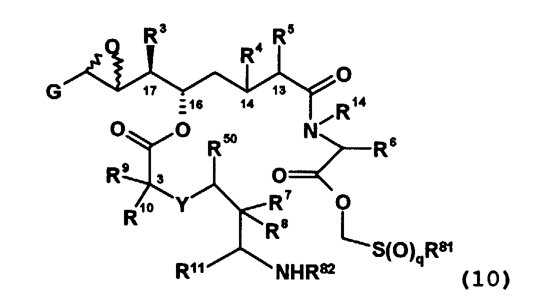 wherein
G is C₁-C₁₂ alkyl, C₂-C₁₂ alkenyl, C₂-C₁₂ alkynyl, or Ar;
Ar is an aromatic or heteroaromatic group or a substituted aromatic or heteroaromatic group;
R³ is C₁-C₆ alkyl;
R⁴ and R⁵ are each hydrogen; or R⁴ and R⁵ taken together form a second bond between C-13 and C-14;
R⁷ and R⁸ are each independently hydrogen or C₁-C₆ alkyl; or
R⁷ and R⁸ taken together form a cyclopropyl or cyclobutyl ring;
R⁹ is hydrogen, C₁-C₆ alkyl, C₂-C₆ alkenyl, C₂-C₆ alkynyl, - (CH₂)ₘ-(C₃-C₅) cycloalkyl or benzyl, wherein m is the integer one to three;
R¹⁰ is hydrogen or C₁-C₆ alkyl;
R¹¹ is hydrogen, C₁-C₆ alkyl, phenyl or benzyl;
R¹⁴ is hydrogen or C₁-C₆ alkyl;
R⁵⁰ is hydrogen or (=O);
Y is CH, O, NR¹², S, SO, SO₂, wherein R¹² is H or C₁-C₃ alkyl;
R⁶ is C₁-C₆ alkyl, substituted (C₁-C₆)alkyl, (C₃-C₈)cycloalkyl, substituted (C₃-C₈)cycloalkyl, a heteroaromatic or substituted heteroaromatic group or a group of formula (IA), (IB) or (IC): 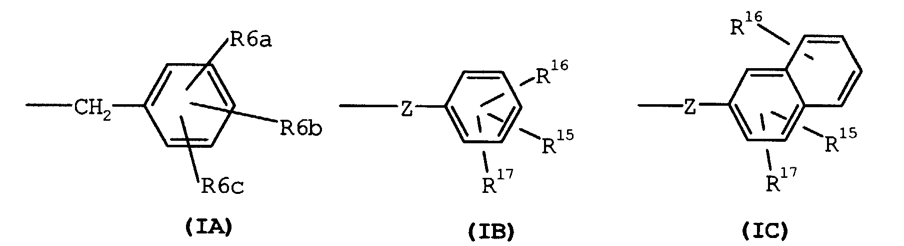
**R**^{**6a**}**, R**^{**6b**}**,** and **R**^{**6c**} independently are H, (C₁-C₆)alkyl, halo NR¹⁸R¹⁹ or OR¹⁸;
**R**^{**15**}**, R**^{**16**}**,** and **R**^{**17**} independently are hydrogen, halo, (C₁-C₆)alkyl, OR¹⁸, O-aryl, NH₂, NR¹⁸R¹⁹, NO₂, OPO₄H₂, (C₁-C₆ alkoxy)phenyl, S-benzyl, CONH₂, C0₂H, PO₃H₂, SO₂R²³, or Z';
**R**^{**18**} and **R**^{**19**} independently are hydrogen or C₁-C₆ alkyl;
**R**^{**23**} is hydrogen or (C₁-C₃)alkyl;
**R**^{**81**} is C₁-C₆ alkyl, C₃-C₈ cycloalkyl, phenyl or benzyl;
**R**^{**82**} is a base labile protecting group;
**Z** is -(CH₂)ₙ- or (C₃-C₅)cycloalkyl;
**n** is 0, 1, or 2;
**q** is an integer 1 or 2; and
**Z'** is an aromatic or substituted aromatic group; or a pharmaceutically acceptable salt thereof.

26. A compound of claim 25 wherein G is phenyl, para-fluorophenyl, or phenyl substituted with -CH₂OC(O) (CH₂)ₘ,NH₂.

27. A compound of claim 25 wherein R³ is methyl.

28. A compound of claim 25 wherein G is phenyl, para-fluorophenyl, or phenyl substituted with -CH₂OC(O) (CH₂)ₘ,NH₂; R³ is methyl; R⁴ and R⁵ taken together form a second bond between C-13 and C-14; R⁹ is C₁-C₆ alkyl; R¹⁰ is hydrogen; R¹¹ is hydrogen; R¹⁴ is hydrogen; R⁵⁰ is (=O); Y is O; and R⁶ is a group of the formula (IA).; R⁸¹ is methyl; R⁸² is Fmoc and q is 1.

29. A compound of claim 25 wherein said compound is represented by the formula

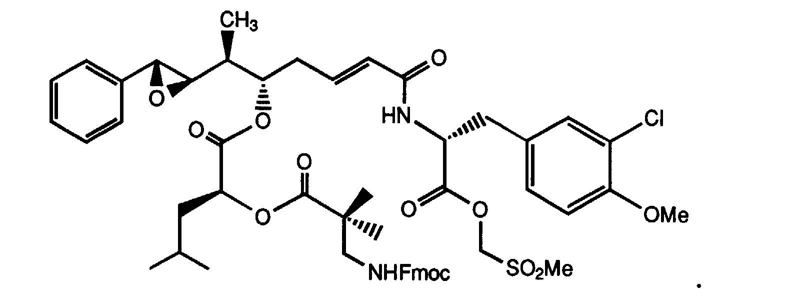

30. A compound of the formula 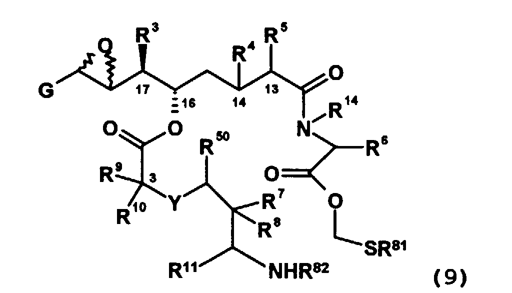 wherein
G is C₁-C₁₂ alkyl, C₂-C₁₂ alkenyl, C₂-C₁₂ alkynyl, or Ar;
Ar is an aromatic or heteroaromatic group or a substituted aromatic or heteroaromatic group;
R³ is C₁-C₆ alkyl;
R⁴ and R⁵ are each hydrogen; or R⁴ and R⁵ taken together form a second bond between C-13 and C-14;
R⁷ and R⁸ are each independently hydrogen or C₁-C₆ alkyl; or
R⁷ and R⁸ taken together form a cyclopropyl or cyclobutyl ring;
R⁹ is hydrogen, C₁-C₆ alkyl, C₂-C₆ alkenyl, C₂-C₆ alkynyl, -(CH₂)ₘ-(C₃-C₅)cycloalkyl or benzyl, wherein m is the integer one to three;
R¹⁰ is hydrogen or C₁-C₆ alkyl;
R¹¹ is hydrogen, C₁-C₆ alkyl, phenyl or benzyl;
R¹⁴ is hydrogen or C₁-C₆ alkyl;
R⁵⁰ is hydrogen or (=O);
Y is CH, O, NH, S, SO, SO₂ or (C₁-C₃)alkylamino;
R⁶ is C₁-C₆ alkyl, substituted (C₁-C₆)alkyl, (C₃-C₈)cycloalkyl, substituted (C₃-C₈)cycloalkyl, a heteroaromatic or substituted heteroaromatic group or a group of formula (IA), (IB) or (IC): 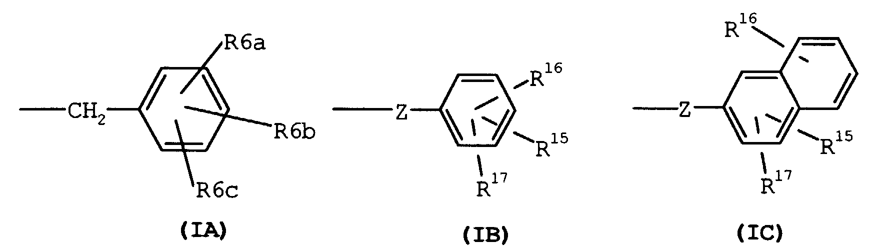
**R**^{**6a**}**, R**^{**6b**}**,** and **R**^{**6c**} independently are H, (C₁-C₆)alkyl, halo NR¹⁸R¹⁹ or OR¹⁸;
**R**^{**15**}**, R**^{**16**}**,** and **R**^{**17**} independently are hydrogen, halo, (C₁-C₆)alkyl, OR¹⁸, O-aryl, NH₂, NR¹⁸R¹⁹, NO₂, OPO₄H₂, (C₁-C₆ alkoxy)phenyl, S-benzyl, CONH₂, C0₂H, PO₃H₂, SO₂R²³, or Z';
**R**^{**18**} and **R**^{**19**} independently are hydrogen or C₁-C₆ alkyl;
**R**^{**23**} is hydrogen or (C₁-C₃)alkyl;
**Z** is -(CH₂)ₙ- or (C₃-C₅)cycloalkyl;
**n** is 0, 1, or 2; and
**Z'** is an aromatic or substituted aromatic group;
R⁸¹ is C₁-C₆ alkyl, C₃-C₈ cyclolkyl, phenyl or benzyl; and
R⁸² is a base labile protecting group; or a pharmaceutically acceptable salt thereof.

31. A compound of claim 30 wherein G is phenyl, para-fluorophenyl, or phenyl substituted with -CH₂OC(O) (CH₂)ₘ,NH₂.

32. A compound of claim 30 wherein R³ is methyl.

33. A compound of claim 30 wherein G is phenyl, para-fluorophenyl, or phenyl substituted with -CH₂OC(O) (CH₂)ₘ,NH₂; R³ is methyl; R⁴ and R⁵ taken together form a second bond between C-13 and C-14; R⁹ is C₁-C₆ alkyl; R¹⁰ is hydrogen; R¹¹ is hydrogen; R¹⁴ is hydrogen; R⁵⁰ is (=O); Y is O; and R⁶ is a group of the formula (IA).; R⁸¹ is methyl; and R⁸² is Fmoc.

34. A compound of claim 30 wherein said compound is represented by the formula

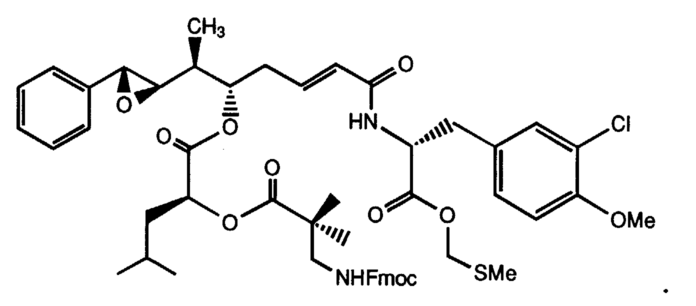

35. A compound of the formula 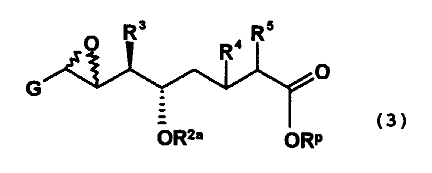 wherein
G is C₁-C₁₂ alkyl, C₂-C₁₂ alkenyl, C₂-C₁₂ alkynyl, or Ar;
Ar is an aromatic or heteroaromatic group or a substituted aromatic or heteroaromatic group;
R³ is C₁-C₆ alkyl;
R⁴ and R⁵ are each hydrogen; or R⁴ and R⁵ taken together form a second bond between C-13 and C-14;
R^{2a} is tri(C₁-C₆ alkyl)silyl; and
R^{p} is hydrogen or a suitable activatable carboxy protecting group, provided that R^{2a} and R^{p} are not both hydrogen; or a pharmaceutically acceptable salt thereof.

36. A compound of claim 35 wherein G is phenyl, para-fluorophenyl, or phenyl substituted with -CH₂OC(O) (CH₂)ₘ,NH₂.

37. A compound of claim 35 wherein R³ is methyl.

38. A compound of claim 35 wherein G is phenyl, para-fluorophenyl, or phenyl substituted with -CH₂OC(O) (CH₂)ₘ,NH₂; R³ is methyl; R⁴ and R⁵ taken together form a second bond between C-13 and C-14; R^{2a} is tertbutyldimethylsilyl; and R^{p} is N-hydroxysuccinimide or hydrogen.

39. A compound of claim 35 wherein said compound is represented by the formula

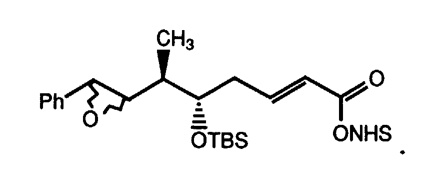

40. A compound of claim 35 wherein said compound is represented by the formula

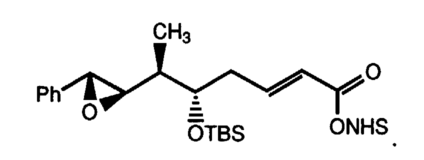

41. A compound of claim 35 wherein said compound is represented by the formula

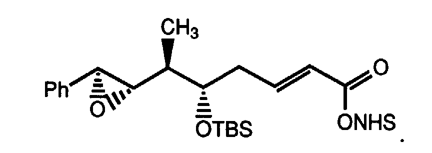

42. A compound of claim 35 wherein said compound is represented by the formula

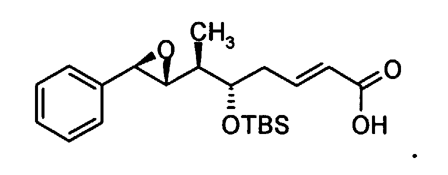

43. A compound of the formula 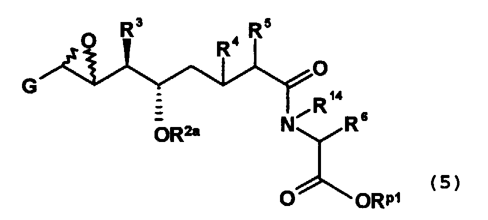 wherein
G is C₁-C₁₂ alkyl, C₂-C₁₂ alkenyl, C₂-C₁₂ alkynyl, or Ar;
Ar is an aromatic or heteroaromatic group or a substituted aromatic or heteroaromatic group;
R³ is C₁-C₆ alkyl;
R⁴ and R⁵ are each hydrogen; or R⁴ and R⁵ taken together form a second bond between C-13 and C-14;
R¹⁴ is hydrogen or C₁-C₆ alkyl;
R⁶ is C₁-C₆ alkyl, substituted (C₁-C₆)alkyl, (C₃-C₈)cycloalkyl, substituted (C₃-C₈)cycloalkyl, a heteroaromatic or substituted heteroaromatic group or a group of formula (IA), (IB) or (IC): 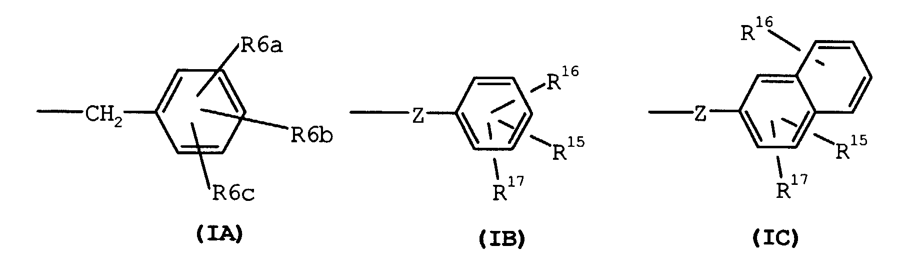
**R**^{**6a**}**, R**^{**6b**}**,** and **R**^{**6c**} independently are H, (C₁-C₆)alkyl, halo NR¹⁸R¹⁹ or OR¹⁸;
**R**^{**15**}**, R**^{**16**}**,** and **R**^{**17**} independently are hydrogen, halo, (C₁-C₆)alkyl, OR¹⁸, O-aryl, NH₂, NR¹⁸R¹⁹, NO₂, OPO₄H₂, (C₁-C₆ alkoxy)phenyl, S-benzyl, CONH₂, C0₂H, PO₃H₂, SO₂R²³, or Z';
**R**^{**18**} and **R**^{**19**} independently are hydrogen or C₁-C₆ alkyl;
**R**^{**23**} is hydrogen or (C₁-C₃)alkyl;
**Z** is -(CH₂)ₙ- or (C₃-C₅)cycloalkyl;
**n** is 0, 1, or 2; and
**Z'** is an aromatic or substituted aromatic group;
**R**^{**p1**} is hydrogen or C₁-C₆ alkyl; and
**R**^{**2a**} is hydrogen or tri(C₁-C₆ alkyl)silyl; or a pharmaceutically acceptable salt thereof.

44. A compound of formula 43 wherein G is phenyl, para-fluorophenyl, or phenyl substituted with -CH₂OC(O) (CH₂)ₘ,NH₂.

45. A compound of claim 43 wherein R³ is methyl.

46. A compound of claim 43 wherein G is phenyl, para-fluorophenyl, or phenyl substituted with -CH₂OC(O) (CH₂)ₘ,NH₂; R³ is methyl; R⁴ and R⁵ taken together form a second bond between C-13 and C-14; R^{2a} is tertbutyldimethylsilyl; and R^{p} is N-hydroxysuccinimide or hydrogen.

47. A compound of claim 43 wherein said compound is represented by the formula

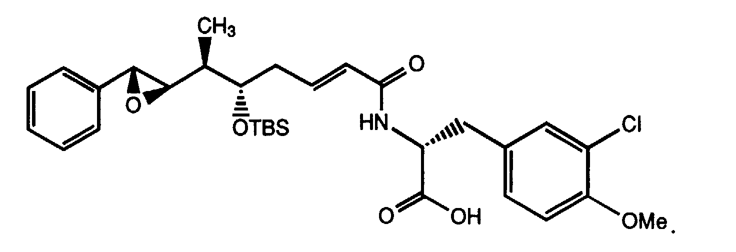

48. A compound of the formula 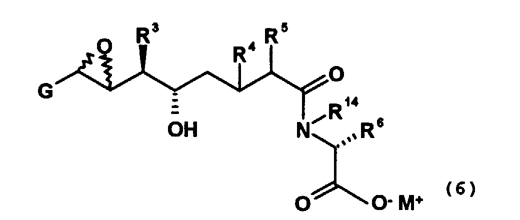 wherein
G is C₁-C₁₂ alkyl, C₂-C₁₂ alkenyl, C₂-C₁₂ alkynyl, or Ar;
Ar is an aromatic or heteroaromatic group or a substituted aromatic or heteroaromatic group;
R³ is C₁-C₆ alkyl;
R⁴ and R⁵ are each hydrogen; or R⁴ and R⁵ taken together form a second bond between C-13 and C-14;
R¹⁴ is hydrogen or C₁-C₆ alkyl;
R⁶ is C₁-C₆ alkyl, substituted (C₁-C₆)alkyl, (C₃-C₈)cycloalkyl, substituted (C₃-C₈)cycloalkyl, a heteroaromatic or substituted heteroaromatic group or a group of formula (IA), (IB) or (IC): 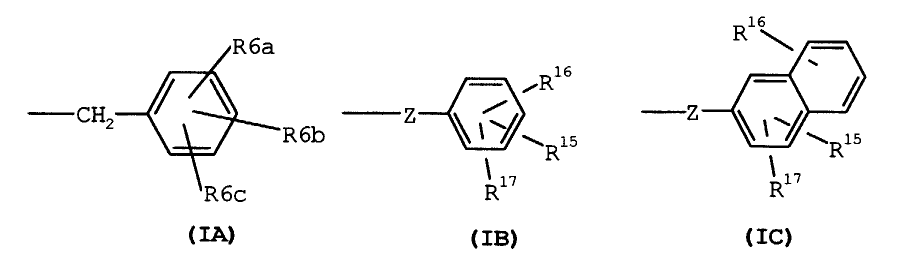
**R**^{**6a**}**, R**^{**6b**}**,** and **R**^{**6c**} independently are H, (C₁-C₆)alkyl, halo NR¹⁸R¹⁹ or OR¹⁸;
**R**^{**15**}**, R**^{**16**}**,** and **R**^{**17**} independently are hydrogen, halo, (C₁-C₆)alkyl, OR¹⁸, O-aryl, NH₂, NR¹⁸R¹⁹, NO₂, OPO₄H₂, (C₁-C₆ alkoxy)phenyl, S-benzyl, CONH₂, C0₂H, PO₃H₂, SO₂R²³, or Z';
**R**^{**18**} and **R**^{**19**} independently are hydrogen or C₁-C₆ alkyl;
**R**^{**23**} is hydrogen or (C₁-C₃)alkyl;
**Z** is -(CH₂)ₙ- or (C₃-C₅)cycloalkyl;
**n** is 0, 1, or 2; and
**Z'** is an aromatic or substituted aromatic group; and
**M**^{**+**} is a cation; or a pharmaceutically acceptable salt thereof.

49. A compound of claim 48 wherein G is phenyl, para-fluorophenyl, or phenyl substituted with -CH₂OC(O) (CH₂)ₘ,NH₂.

50. A compound of claim 48 wherein R³ is methyl.

51. A compound of claim 48 wherein G is phenyl, para-fluorophenyl, or phenyl substituted with -CH₂OC(O) (CH₂)ₘ,NH₂; R³ is methyl; R⁴ and R⁵ taken together form a second bond between C-13 and C-14; R¹⁴ is hydrogen; R⁶ is a group of the formula (IA); and R^{p1} hydrogen or methyl.

52. A compound of claim 48 wherein said compound is represented by the formula

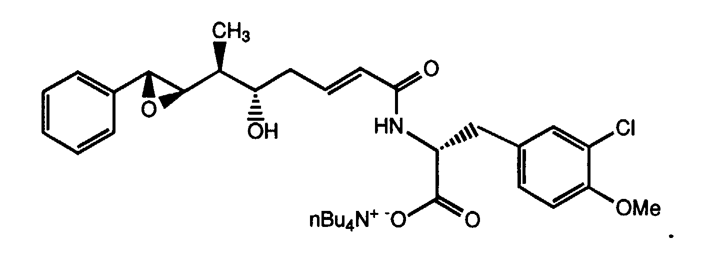

53. A compound of claim 48 wherein said compound is represented by the formula

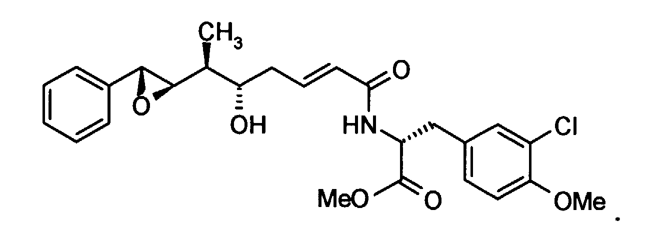

54. A compound of the formula 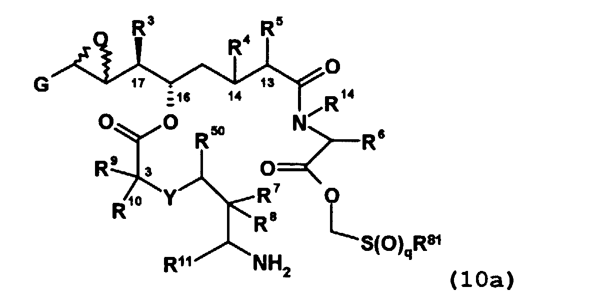 wherein
G is C₁-C₁₂ alkyl, C₂-C₁₂ alkenyl, C₂-C₁₂ alkynyl, or Ar;
Ar is an aromatic or heteroaromatic group or a substituted aromatic or heteroaromatic group;
R³ is C₁-C₆ alkyl;
R⁴ and R⁵ are each hydrogen; or R⁴ and R⁵ taken together form a second bond between C-13 and C-14;
R⁷ and R⁸ are each independently hydrogen or C₁-C₆ alkyl; or
R⁷ and R⁸ taken together form a cyclopropyl or cyclobutyl ring;
R⁹ is hydrogen, C₁-C₆ alkyl, C₂-C₆ alkenyl, C₂-C₆ alkynyl, -(CH₂)ₘ-(C₃-C₅)cycloalkyl or benzyl, wherein m is the integer one to three;
R¹⁰ is hydrogen or C₁-C₆ alkyl;
R¹¹ is hydrogen, C₁-C₆ alkyl, phenyl or benzyl;
R¹⁴ is hydrogen or C₁-C₆ alkyl;
R⁵⁰ is hydrogen or (=O);
Y is CH, O, NR¹², S, SO, SO₂, wherein R¹² is H or C₁-C₃ alkyl;
R⁶ is C₁-C₆ alkyl, substituted (C₁-C₆)alkyl, (C₃-C₈)cycloalkyl, substituted (C₃-C₈)cycloalkyl, a heteroaromatic or substituted heteroaromatic group or a group of formula (IA), (IB) or (IC): 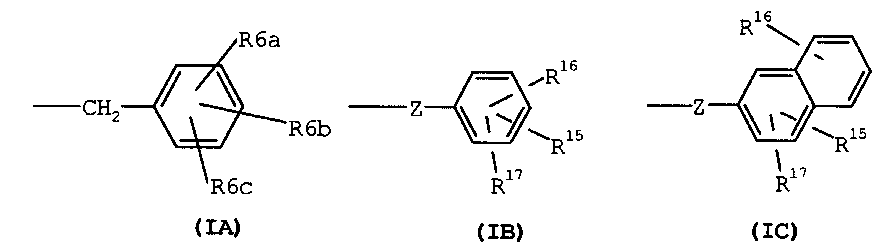
**R**^{**6a**}**, R**^{**6b**}**,** and **R**^{**6c**} independently are H, (C₁-C₆)alkyl, halo NR¹⁸R¹⁹ or OR¹⁸;
**R**^{**15**}**, R**^{**16**}**,** and **R**^{**17**} independently are hydrogen, halo, (C₁-C₆)alkyl, OR¹⁸, O-aryl, NH₂, NR¹⁸R¹⁹, NO₂, OPO₄H₂, (C₁-C₆ alkoxy)phenyl, S-benzyl, CONH₂, C0₂H, PO₃H₂, SO₂R²³, or Z';
**R**^{**18**} and **R**^{**19**} independently are hydrogen or C₁-C₆ alkyl;
**R**^{**23**} is hydrogen or (C₁-C₃)alkyl;
**R**^{**81**} is C₁-C₆ alkyl, C₃-C₈ cycloalkyl, phenyl or benzyl;
**Z** is -(CH₂)ₙ- or (C₃-C₅)cycloalkyl;
**n** is 0, 1, or 2;
**q** is an integer 1 or 2; and
**Z'** is an aromatic or substituted aromatic group; or a pharmaceutically acceptable salt thereof.

55. A compound of claim 54 wherein G is phenyl, para-fluorophenyl, or phenyl substituted with -CH₂OC(O) (CH₂)ₘ,NH₂.

56. A compound of claim 54 wherein R³ is methyl.

57. A compound of claim 54 wherein G is phenyl, para-fluorophenyl, or phenyl substituted with -CH₂OC(O) (CH₂)ₘ,NH₂; R³ is methyl; R⁴ and R⁵ taken together form a second bond between C-13 and C-14; R^{p} is NHS or hydrogen; and R^{2a} is tri(C₁-C₆ alkyl)silyl.

58. A compound of claim 54 represented by the formula

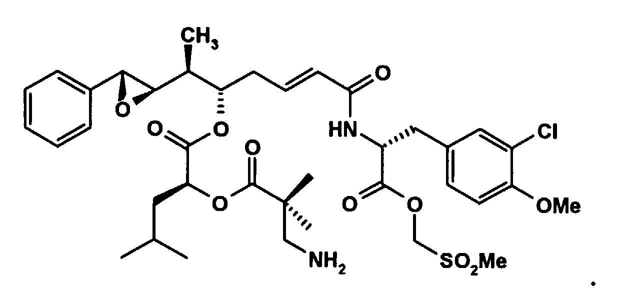

59. A compound of the formula 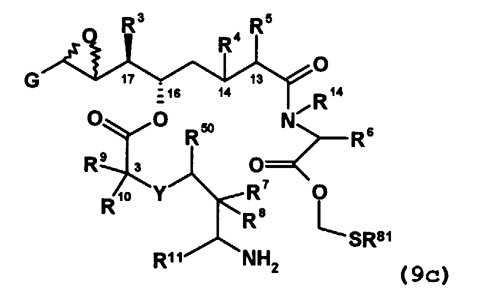 wherein
G is C₁-C₁₂ alkyl, C₂-C₁₂ alkenyl, C₂-C₁₂ alkynyl, or Ar;
Ar is an aromatic or heteroaromatic group or a substituted aromatic or heteroaromatic group;
R³ is C₁-C₆ alkyl;
R⁴ and R⁵ are each hydrogen; or R⁴ and R⁵ taken together form a second bond between C-13 and C-14;
R⁷ and R⁸ are each independently hydrogen or C₁-C₆ alkyl; or
R⁷ and R⁸ taken together form a cyclopropyl or cyclobutyl ring;
R⁹ is hydrogen, C₁-C₆ alkyl, C₂-C₆ alkenyl, C₂-C₆ alkynyl, -(CH₂)ₘ-(C₃-C₅)cycloalkyl or benzyl, wherein m is the integer one to three;
R¹⁰ is hydrogen or C₁-C₆ alkyl;
R¹¹ is hydrogen, C₁-C₆ alkyl, phenyl or benzyl;
R¹⁴ is hydrogen or C₁-C₆ alkyl;
R⁵⁰ is hydrogen or (=O);
Y is CH, O, NR¹², S, SO, SO₂, wherein R¹² is H or C₁-C₃ alkyl;
R⁶ is C₁-C₆ alkyl, substituted (C₁-C₆)alkyl, (C₃-C₈)cycloalkyl, substituted (C₃-C₈)cycloalkyl, a heteroaromatic or substituted heteroaromatic group or a group of formula (IA), (IB) or (IC): 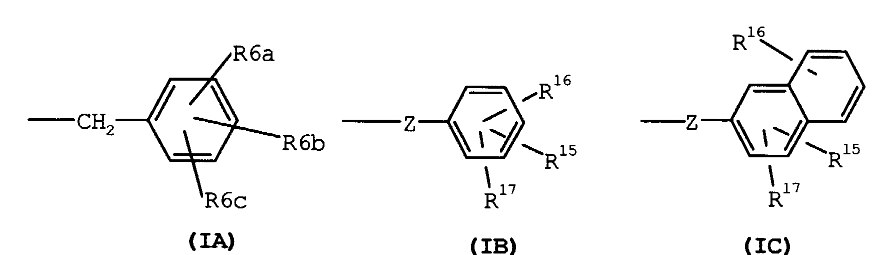
**R**^{**6a**}**, R**^{**6b**}**,** and **R**^{**6c**} independently are H, (C₁-C₆)alkyl, halo NR¹⁸R¹⁹ or OR¹⁸;
**R**^{**15**}**, R**^{**16**}**,** and **R**^{**17**} independently are hydrogen, halo, (C₁-C₆)alkyl, OR¹⁸, O-aryl, NH₂, NR¹⁸R¹⁹, NO₂, OPO₄H₂, (C₁-C₆ alkoxy)phenyl, S-benzyl, CONH₂, C0₂H, PO₃H₂, SO₂R²³, or Z';
**R**^{**18**} and **R**^{**19**} independently are hydrogen or C₁-C₆ alkyl;
**R**^{**23**} is hydrogen or (C₁-C₃)alkyl;
**R**^{**81**} is C₁-C₆ alkyl, C₃-C₈ cycloalkyl, phenyl or benzyl;
**Z** is -(CH₂)ₙ- or (C₃-C₅)cycloalkyl;
**n** is 0, 1, or 2; and
**Z'** is an aromatic or substituted aromatic group; or a pharmaceutically acceptable salt thereof.

60. A compound of claim 59 wherein G is phenyl, para-fluorophenyl, or phenyl substituted with -CH₂OC(O) (CH₂)ₘ,NH₂.

61. A compound of claim 59 wherein R³ is methyl.

62. A compound of claim 59 wherein G is phenyl, para-fluorophenyl, or phenyl substituted with -CH₂OC(O) (CH₂)ₘ,NH₂; R³ is methyl; R⁴ and R⁵ taken together form a second bond between C-13 and C-14; R^{p} is NHS or hydrogen; and R^{2a} is tri(C₁-C₆ alkyl)silyl.

63. A compound of claim 59 represented by the formula

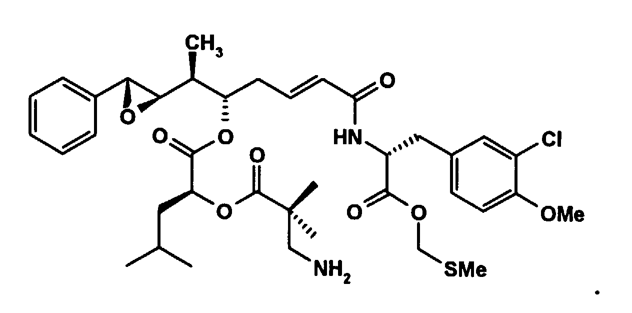

64. A compound of the formula 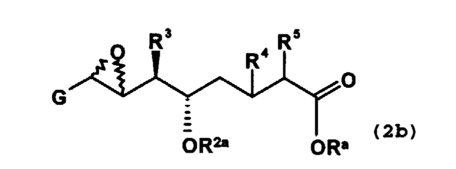
wherein
G is C₁-C₁₂ alkyl, C₂-C₁₂ alkenyl, C₂-C₁₂ alkynyl, or Ar;
Ar is an aromatic or heteroaromatic group or a substituted aromatic or heteroaromatic group;
R³ is C₁-C₆ alkyl;
R⁴ and R⁵ are each hydrogen; or R⁴ and R⁵ taken together form a second bond between C-13 and C-14;
R^{2a} is tri(C₁-C₆ alkyl)silyl; and
R^{a} is C₁-C₆ alkyl; or a pharmaceutically acceptable salt thereof.

65. A compound of claim 64 wherein G is phenyl, para-fluorophenyl, or phenyl substituted with -CH₂OC(O) (CH₂)ₘ,NH₂.

66. A compound of claim 64 wherein R³ is methyl.

67. A compound of claim 64 wherein G is phenyl, para-fluorophenyl, or phenyl substituted with -CH₂OC(O) (CH₂)ₘ,NH₂; R³ is methyl; R⁴ and R⁵ taken together form a second bond between C-13 and C-14; R^{2a} is tertbutyldimethylsilyl; and R^{a} is methyl.

68. A compound of claim 64 wherein said compound is represented by the formula

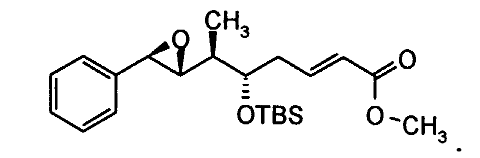

69. A compound of the formula 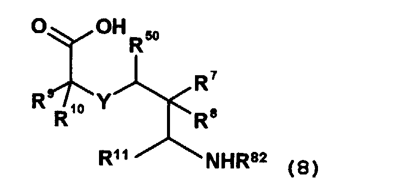 wherein
R⁷ and R⁸ are each independently hydrogen or C₁-C₆ alkyl; or
R⁷ and R⁸ taken together form a cyclopropyl or cyclobutyl ring;
R⁹ is hydrogen, C₁-C₆ alkyl, C₂-C₆ alkenyl, C₂-C₆ alkynyl, -(CH₂)ₘ-(C₃-C₅)cycloalkyl or benzyl, wherein m is the integer one to three;
R¹⁰ is hydrogen or C₁-C₆ alkyl;
R¹¹ is hydrogen, C₁-C₆ alkyl, phenyl or benzyl;
R⁵⁰ is hydrogen or (=O);
Y is CH, O, NR¹², S, SO, SO₂, wherein R¹² is H or C₁-C₃ alkyl;
**R**^{**82**} is a base labile protecting group;
or a pharmaceutically acceptable salt thereof.

70. A compound of claim 69 wherein R⁹ is C₁-C₆ alkyl; R¹⁰ is hydrogen; R¹¹ is hydrogen; R⁵⁰ is (=O); Y is O; and R⁸² is Fmoc.

71. A compound of claim 69 wherein said compound is represented by the formula
